# EUROPÄISCHE PATENTANMELDUNG

(11) **EP 1 116 484 A2**
(43) Veröffentlichungstag der Anmeldung: **18.07.2001**
(21) Anmeldenummer: 00127655.9
(22) Anmeldetag: 18.12.2000
(51) Int. Cl.: A61K 7/06, A61K 7/48

(54) **Verfahren zur Behandlung eines kosmetischen Mittels durch Bestrahlung mit NIR-Strahlung, sowie dessen Verwendung**

(30) Priorität: 12.01.2000 DE 10000807
(71) Anmelder: BASF AKTIENGESELLSCHAFT, 67056 Ludwigshafen (DE)
(72) Erfinder: Witteler, Helmut, Dr., 67259 Beindersheim (DE); Blum, Rainer, 67069 Ludwigshafen (DE); Hössel, Peter, Dr., 67105 Schifferstadt (DE); Sanner, Axel, Dr., 67227 Frankenthal (DE); Schwalm, Reinhold, Dr., 67157 Wachenheim (DE); Dausch, Wilma M., Dr., 67117 Limburgerhof (DE); Jaworek, Thomas, Dr., 67169 Kallstadt (DE); Königer, Rainer, Dr., 67251 Freinsheim (DE)

(57) **Zusammenfassung**

Die vorliegende Erfindung betrifft ein Verfahren zur Behandlung eines kosmetischen Mittels, dadurch gekennzeichnet, daß das kosmetische Mittel vor, während oder nach der Applikation mit NIR-Strahlung behandelt wird.

## Beschreibung

Die vorliegende Erfindung betrifft ein Verfahren zur Behandlung eines kosmetischen Mittels vor, während oder nach der Applikation durch Bestrahlung mit NIR-Strahlung, sowie die Verwendung des so erhaltenen kosmetischen Mittels zur Behandlung der Haut, Haare oder der Nägel. Bevorzugt wird das erfindungsgemäße kosmetische Mittel zur Festigung, Strukturverbesserung und Formgebung der Haare verwendet.

Ein weiterer Gegenstand der Erfindung ist ein kosmetisches Mittel, erhältlich durch NIR-Bestrahlung.

Verfahren zur Veränderung der Eigenschaften von Kosmetika während der Applikation durch Einwirkung von Wärme, UV-Strahlung oder reaktiven Chemikalien sind im Stand der Technik vielfach beschrieben.

U.S. 3,820,550 beschreibt die Aushärtung N-substituierter Acrylamide, insbesondere von Diacetonacrylamiden auf dem Haar durch Hitze, Redox-Katalysatoren oder Autoxidation.

U.S. 3,472,604 beschreibt die Behandlung des Haares beim Färben mit wasserlöslichen polymerisierbaren Vinylmonomeren, wobei das Monomer mittels eines Oxidationsmittels polymerisiert wird.

U.S. 3,634,022 und U.S. 3,676,550 beschreiben ein Verfahren zur Applikation von Dauerwellen, wobei neben üblichen Methoden zusätzlich olefinisch ungesättigte Monomere oder Peroxid-Initiatoren verwendet werden.

U.S. 4,278,659 beschreibt Haarbehandlungsmittel, enthaltend eine Säure, Glyceraldehyd, Resorcinol sowie ein oligomeres Präkondensat davon, welche mittels Hitze appliziert werden.

In U.S. 5,362,486 wird die Polymerisierung von Urethan-Acrylat Oligomeren auf dem Haar beschrieben, wobei als Polymerisierungsreagentien Benzoyl-Peroxid und andere eingesetzt werden.

U.S. 4,682,612 beschreibt ein Verfahren zur Herstellung künstlicher Nägel, die durch UVA-Strahlung (320 bis 400 nm) ausgehärtet werden.

Allen diesen Verfahren ist gemeinsam, daß sie mit Methoden arbeiten, die für den Kontakt mit dem menschlichen Körper ungünstig sind, weil sie zu Hautirritationen und zu gesundheitlichen Schäden führen können. Ein weiterer Nachteil besteht bei einigen der genannten Verfahren darin, daß die Aushärtung der verwendeten Polymere oder Präpolymere hinsichtlich ihres Zeitpunktes und ihrer Dauer nicht genau steuerbar ist. Ferner ist es ein Nachteil der Behandlung mit UV-Licht und Wärmestrahlung, daß diese nicht durch das Haar hindurchtritt, und somit keine durchdringende und zeitlich optimierte Behandlung des auf dem Haar vorliegenden kosmetischen Mittels möglich ist.

Die gezielte Beeinflussung kosmetischer Präparate durch Einwirkung von NIR-Strahlung zu deren Herstellung oder Anwendung ist bislang unbekannt. Insbesondere die Anwendung von kosmetischen Formulierungen zusammen mit NIR-Strahlung, so daß bei der Anwendung Polymere entstehen oder vernetzen oder die Eigenschaften von Polymeren verändert werden, ist nicht bekannt.

Der vorliegenden Erfindung lag daher die Aufgabe zugrunde, ein Verfahren zur Behandlung kosmetischer Formulierungen bereitzustellen, das eine verbesserte Haar- und Hautpflege ermöglicht und das die oben genannten Nachteile nicht aufweist.

Überraschenderweise wurde nun gefunden, daß es gelingt mit Hilfe von Nah-Infra-Rot (NIR)-Strahlung praktisch jedes zur Polymerisation, Härtung oder Vernetzung geeignete System wirksam zu machen, also auch solche Systeme, die im allgemeinen nur mit Hilfe von UV-Strahlung oder langwelliger Infrarotstrahlung gehärtet werden. Dies ist umso überraschender, weil die unten genannten Monomere, Präpolymere und Polymere UV-Strahlung und langwellige Infrarot-Strahlung viel stärker absorbieren als NIR-Strahlung.

Der besondere Vorteil der Erfindung gegenüber der Aktivierung oder Polymerisation kosmetischer Mittel durch Wärme, UV-Strahlung, Behandlung mit Radikalbildnern, Säuren und Basen besteht in der vereinfachten Handhabung und in der signifikant verringerten Irritation der betroffenen Körperpartien. Gegenüber IR-Strahlung besteht der Vorteil, daß NIR-Strahlung tiefer als IR-Strahlung in das kosmetische Mittel eindringt und somit nicht nur oberflächlich wirkt. Das ist insbesondere dann von Vorteil, wenn das kosmetische Mittel erst nach dem Aufbringen oder Eindringen in das Substrat (z.B. Haut oder Haar) aktiviert, gehärtet oder polymerisiert werden soll. Im Gegensatz zu IR-Strahlung tritt NIR-Strahlung außerdem durch das Haar mit wesentlich schwächerer Absorption hindurch, so daß NIR-Strahlung gerade für voluminöse Frisuren geeignet ist.

Ein erster Gegenstand der Erfindung ist ein Verfahren zur Behandlung eines kosmetischen Mittels, dadurch gekennzeichnet, daß das kosmetische Mittel vor, während oder nach der Applikation mit Nah-Infra-Rot-Strahlung (NIR-Strahlung) behandelt wird.

Ein weiterer Gegenstand der Erfindung ist ein kosmetisches Mittel, erhältlich durch die Bestrahlung mit NIR-Strahlung sowie die Verwendung des so erhaltenen Mittels zur Behandlung der Haut, der Haare oder der Nägel.

Bei den kosmetischen Mitteln handelt es sich insbesondere um Mittel mit filmbildender, permeationshemmender, festigender oder formgebender Wirkung, deren Wirkung durch NIR-Strahlung mit einem Emissionsmaximum im Bereich der Wellenlängen 600 nm bis 1500 nm, insbesondere 750 bis 1100 nm während oder nach der Applikation, aktiviert, verstärkt oder beschleunigt wird.

Das kosmetische Mittel ist dadurch gekennzeichnet, daß unter Einwirkung von NIR-Strahlung mindestens ein Polymer gebildet wird, wobei das Polymer linear, verzweigt oder vernetzt sein kann; auch die Bildung von statistischen und alternierenden Copolymeren sowie von Pfropf- und Blockcopolymeren ist möglich. Die Bildung des Polymers erfolgt aus beliebigen in dem kosmetischen Mittel enthaltenen chemischen Verbindungen, wobei organische und siliciumorganische Verbindungen bevorzugt sind. Besonders bevorzugt sind Verbindungen, die zur chemischen Reaktion unter Polymerisation, Polyaddition, Polykondensation oder Vernetzung befähigt sind oder unter Kombination dieser Reaktionstypen reagieren. Die Reaktion kann auch unter chemischer Modifikation der behandelten Struktur (Haar, Nägel, keratinisches Material, Haut) ablaufen. Insbesondere enthält das kosmetische Mittel Verbindungen aus den folgenden Klassen:
- ungesättigte Verbindungen (insbesondere ethylenisch ungesättigte Verbindungen),
- Epoxide
- Siliciumverbindungen mit wenigstens einer Si-O-Bindung und gegebenenfalls wenigstens einer Si-C-Bindung
- Aromaten, die wenigstens eine C-O-Bindung unter Beteiligung eines Ringatoms aufweisen, beispielsweise Phenolabkömmlinge
- Formaldehyd und Verbindungen die Formaldehyd abspalten können
- Urotropin, Melamin, Harnstoff sowie Derivate oder strukturelle Abkömmlinge davon
- Stoffe, die unter geeigneten Bedingungen in Radikale zerfallen
- Ferner kann das kosmetische Mittel Isocyanate, Alkohole, Amine und ihre Derivate, Carbonsäuren und ihre Derivate, Lactame und Lactone, Carbodiimide enthalten.

Alle Verbindungen aus den genannten Klassen können niedermolekularen oder hochmolekularen Charakter haben. Ausschlaggebend ist alleine ihre Fähigkeit, durch chemische Reaktion Substanzen höheren Molekulargewichts oder höherer mechanischer Festigkeit oder niedrigerer Permeabilität (für Wasser oder Sauerstoff) zu bilden, oder durch chemische Reaktion mit der behandelten Struktur eine entsprechende Veränderung der Eigenschaften der behandelten Struktur zu bewirken. Polymere, die unter geeigneten Bedingungen vernetzen, sind ebenfalls als Bestandteil der kosmetischen Formulierungen geeignet.

Das erfindungsgemäß verwendete kosmetische Mittel dient bevorzugt zur Behandlung von Haut, Haar und Nägeln, wobei die Behandlung des Haars mit festigender oder fixierender Wirkung bevorzugt ist.

Besonders geeignete Strahlungsquellen für NIR-Strahlung emittieren überwiegend, wenigstens aber stärker als handelsübliche Infrarot-Strahler, im Wellenlängenbereich von 600 nm bis 1500 nm. NIR-Strahler können beispielsweise Halogen-Lampen, Halbleiterlaser, Leuchtdioden, Quecksilberdampflampen oder Natriumdampf-Lampen sein. Handelsübliche NIR-Strahler werden beispielsweise von den Firmen IndustrieServis (Bruckmühl) und Ushio (Tokyo) angeboten.

Erfindungsgemäß sind insbesondere Verfahren, bei denen NIR-Strahlung zusammen mit einer Formulierung in der Haarkosmetik zum Einsatz kommt, vorzugsweise mit Zubereitungen wie Haarfestiger-Formulierungen, Dauerwellpräparaten, Haarkuren, Haarlotionen, Haarspülungen, Haaremulsionen, Spitzenfluids, Egalisierungsmittel für Dauerwellen, Hot-Oil-Treatment-Präparate, Conditioner, Festigerlotionen, Shampoos, Haarfärbemittel, Mittel zur Behandlung von Schuppen und Haarausfall sowie in Haarwuchsmitteln. Das Verfahren wird bevorzugt zur Festigung der Haare eingesetzt. Erfindungsgemäß ist sowohl der Einsatz in typischen Haarfestigerformulierungen wie auch die Kombination mit anderen Methoden und Mitteln zur Verformung der Haare, besonders mit Dauerwellen.

Ferner ist das erfindungsgemäße Verfahren auf die Nägel anwendbar, insbesondere in Verbindung mit der Verwendung von Nagellacken.

Das erfindungsgemäße Verfahren kann außerdem für kosmetische und dermatologische Mittel für die Haut eingesetzt werden. Anwendungszweck sind hier insbesondere Hautpräparate mit permeationshemmender Wirkung (Permeation von Wasser) und Präparate zur Wundversorgung.

Außerdem wird das erfindungsgemäße Verfahren für kosmetische Mittel zur Reinigung der Haut verwendet. Solche kosmetischen Reinigungsmittel sind ausgewählt aus Stückseifen, wie Toilettenseifen, Kernseifen, Transparentseifen, Luxusseifen, Deoseifen, Cremeseifen, Babyseifen, Hautschutzseifen, Abrasiveseifen und Syndets, flüssigen Seifen, wie pastöse Seifen, Schmierseifen und Waschpasten, und flüssigen Wasch-, Dusch-, und Badepräparaten, wie Waschlotionen, Duschbädern und -gelen, Schaumbädern, Ölbädern und Scrub-Präparaten.

Weiterhin kann das Verfahren für kosmetische Mittel zur Pflege und zum Schutz der Haut, in Nagelpflegemitteln sowie in Zubereitungen für die dekorative Kosmetik angewendet werden.

Eine weitere Anwendung des Verfahrens ist die Verwendung bei Hautpflegemitteln, Intimpflegemitteln, Fußpflegemitteln, Lichtschutzmitteln, Repellents, Rasiermitteln, Haarentfernungsmitteln, Antiaknemitteln, Make-up, Maskara, Lippenstifte, Lidschatten, Kajalstiften, Eyelinern, Rouges, Pudern und Augenbrauenstiften.

Die Hautpflegemittel liegen insbesondere als W/O- oder O/W-Hautcremes, Tag- und Nachtcremes, Augencremes, Gesichtscremes, Antifaltencremes, Feuchthaltecremes, Bleichcremes, Vitamincremes, Hautlotionen, Pflegelotionen und Feuchthaltelotionen vor.

Außerdem eignet sie das Verfahren für Nose-Strips zur Porenreinigung, Antiaknemitteln, Repellents, Rasiermitteln, Haarentfernungsmitteln, Intimpflegemitteln, Fußpflegemitteln sowie in der Babypflege. Ebenso geeignet ist das Verfahren für die Applikation von Hilfsmitteln in der Pharmazie und Galenik, bevorzugt für die Verwendung von Beschichtungsmitteln und Bindemitteln für feste Arzneiformen.

Je nach Anwendungsgebiet können die erfindungsgemäßen Mittel in einer zur Hautpflege geeigneten Form, wie z.B. als Creme, Schaum, Gel, Gelspray, Stift, Pulver, Mousse, Milch oder Lotion appliziert werden.

Bevorzugt wird das Verfahren in kosmetischen Mitteln, enthaltend die folgenden Klassen von Monomeren, Präpolymeren und Polymeren eingesetzt:

Ethylenisch ungesättigte Monomere, die mit einer durch freie Radikale initiierten Reaktion polymerisiert werden können, sind bevorzugt. Der Begriff ethylenisch ungesättigt bedeutet, daß die Monomere zumindest eine polymerisierbare Kohlenstoff-Kohlenstoff Doppelbindung besitzen, die mono-, di-, tri-, oder tetrasubstituiert sein kann.

Die bevorzugten ethylenisch ungesättigten Monomere können durch die folgende allgemeine Formel beschrieben werden:

X-C (O)CR⁷=CHR⁶

wobei
X ausgewählt ist aus der Gruppe der Reste -OH, -OM, -OR⁸, NH2, -NHR⁸, N(R⁸)₂ ;
M ist ein Kation ausgewählt aus der Gruppe bestehend aus: Na⁺, K⁺, Mg⁺⁺, Ca⁺⁺, Zn⁺⁺, NH⁴⁺, Alkylammonium, Dialkylammonium, Trialkylammonium und Tetraalkylammonium;
die Reste R⁸ können identisch oder verschieden ausgewählt werden aus der Gruppe bestehend aus -H, C₁-C₄₀ linear- oder verzweigtkettige Alkylreste, N,N-Dimethylaminoethyl, 2-Hydroxyethyl, 2-Methoxyethyl, 2-Ethoxyethyl, Hydroxypropyl, Methoxypropyl oder Ethoxypropyl.

R⁷ und R⁶ sind unabhängig voneinander ausgewählt aus der Gruppe bestehend aus: -H, C₁-C₈ linear- oder verzweigtkettige Alkylketten, Methoxy, Ethoxy, 2-Hydroxyethoxy, 2-Methoxyethoxy und 2-Ethoxyethyl.

Repräsentative aber nicht limitierende Beispiele von geeigneten Monomeren sind zum Beispiel Acrylsäure und deren Salze, Ester und Amide. Die Salze können von jedem beliebigen nicht toxischen Metall, Ammonium oder substituierten Ammonium-Gegenionen abgeleitet sein.

Die Ester können abgeleitet sein von C₁-C₄₀ linearen, C₃-C₄₀ verzweigtkettigen, oder C₃-C₄₀ carbocyclischen Alkoholen, von mehrfachfunktionellen Alkoholen mit 2 bis etwa 8 Hydroxylgruppen wie Ethylenglycol, Hexylenglycol, Glycerin, and 1,2,6-Hexantriol, von Aminoalkoholen oder von Alkoholethern wie Methoxyethanol und Ethoxyethanol oder Polyethylenglykolen.

Ferner eignen sich N,N-Dialkylaminoalkylacrylate- und methacrylate und N-Dialkylaminoalkylacryl- und -methacrylamide der allgemeinen Formel (II)
- mit R⁹: = H, Alkyl mit 1 bis 8 C-Atomen,
- R¹⁰: = H, Methyl,
- R¹¹: = Alkylen mit 1 bis 24 C-Atomen, optional substituiert durch Alkyl,
- R¹², R¹³: = C₁-C₄₀ Alkylrest,
- Z: = Stickstoff für x = 1 oder Sauerstoff für x = 0

Die Amide können unsubstituiert, N-Alkyl oder N-alkylamino monosubstituiert, oder N,N-dialkylsubstituiert oder N,N-dialkylamino disubstituiert, worin die Alkyl- oder Alkylaminogruppen von C₁-C₄₀ linearen, C₃-C₄₀ verzweigtkettigen, oder C₃-C₄₀ carbocyclischen Einheiten abgeleitet sind. Zusätzlich können die Alkylaminogruppen quarternisiert werden.

Bevorzugte Monomere der Formel II sind N,N-Dimethylaminomethyl(meth)acrylat, N,N-Diethylaminomethyl (meth)acrylat, N,N-Dimethylaminoethyl(meth)acrylat, N,N-Diethylaminoethyl (meth)acrylat.

Ein ebenfalls verwendbares Monomer ist Diacetonacrylamid (CAS-Nummer 2873-97-4).

Ebenfalls verwendbare Monomere sind substituierte Acrylsäuren sowie Salze, Ester und Amide davon, wobei die Substituenten an den Kohlenstoffatomen in der zwei oder drei Position der Acrylsäure stehen, und unabhängig voneinander ausgewählt sind aus der Gruppe bestehend aus C₁-C₄ Alkyl, -CN, COOH besonders bevorzugt Methacrylsäure, Ethacrylsäure und 3-Cyanoacrylsäure. Diese Salze, Ester und Amide dieser substituierten Acrylsäuren können wie oben für die Salze, Ester und Amide der Acrylsäure beschrieben ausgewählt werden.

Andere geeignete Monomere sind Vinyl- und Allylester von C₁-C₄₀ linearen, C₃-C₄₀ verzweigtkettigen oder C₃-C₄₀ carbocyclische Carbonsäuren (z.B.: Vinylacetat, Vinylpropionat, Vinylneononanoat, Vinylneoundekansäure oder t-Butyl-benzoesäure-vinylester); Vinyl- oder Allylhalogenide, bevorzugt Vinylchlorid und Allylchlorid, Vinylether, bevorzugt Methyl-, Ethyl-, Butyl-, oder Dodecylvinylether, Vinylformamid, Vinylmethylacetamid, Vinylamin; Vinyllactame, bevorzugt Vinylpyrrolidon und Vinylcaprolactam, Vinyl- oder Allyl-substituierte heterocyclische Verbindungen, bevorzugt Vinylpyridin, Vinyloxazolin und Allylpyridin.

Weiterhin sind N-Vinylimidazole der allgemeinen Formel III geeignet, worin R¹⁴ bis R¹⁶ unabhängig voneinander für Wasserstoff, C₁-C₄-Alkyl oder Phenyl steht:

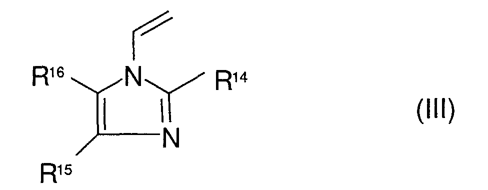

Weitere geeignete Monomere sind Diallylamine der allgemeinen Formel (IV) 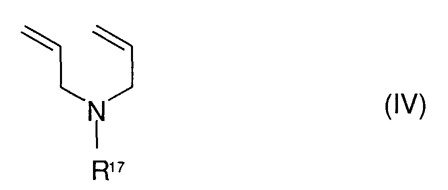 mit R¹⁷ = C₁-C₂₄ Alkyl

Weitere geeignete Monomere sind Vinylidenchlorid; und Kohlenwasserstoffe mit mindestens einer Kohlenstoff-Kohlenstoff Doppelbindung, bevorzugt Styrol, alpha-Methylstyrol, tert.-Butylstyrol, Butadien, Isopren, Cyclohexadien, Ethylen, Propylen, 1-Buten, 2-Buten, Isobutylen, Vinyltoluol, sowie Mischungen dieser Monomere.

Besonders geeignete Monomere sind Acrylsäure, Methacrylsäure, Ethylacrylsäure, Methylacrylat, Ethylacrylat, Propylacrylat, n-Butylacrylat, iso-Butylacrylat, t-Butylacrylat, 2-Ethylhexylacrylat, Decylacrylat, Methylmethacrylat, Ethylmethacrylat, Propylmethacrylat, n-Butylmethacrylat, iso-Butylmethacrylat, t-Butylmethacrylat, 2-Ethylhexylmethacrylat, Decylmethacrylat, Methylethacrylat, Ethylethacrylat, n-Butylethacrylat, iso-Butylethacrylat, t-Butylethacrylat, 2-Ethylhexylethacrylat, Decylethacrylat, 2,3-Dihydroxypropylacrylat, 2,3-Dihydroxypropylmethacrylat, 2-Hydroxyethylacrylat, Hydroxypropylacrylate, 2-Hydroxyethylmethacrylat, 2-Hydroxyethylethacrylat, 2-Methoxyethylacrylat, 2-Methoxyethylmethacrylat, 2-Methoxyethylethacrylat, 2-Ethoxyethylmethacrylat, 2-Ethoxyethylethacrylat, Hydroxypropylmethacrylate, Glycerylmonoacrylat, Glycerylmonomethacrylat, Polyalkylenglykol (meth) acrylate, ungesättigte Sulfonsäuren wie zum Beispiel Acrylamidopropansulfonsäure;

Acrylamid, Methacrylamid, Ethacrylamid, N-Methylacrylamid, N,N-Dimethylacrylamid, N-Ethylacrylamid, N-Isopropylacrylamid, N-Butylacrylamid, N-t-Butylacrylamid, N-Octylacrylamid, N-t-Octylacrylamid, N-Octadecylacrylamid, N-Phenylacrylamid, N-Methylmethacrylamid, N-Ethylmethacrylamid, N-Dodecylmethacrylamid, 1-Vinylimidazol, 1-Vinyl-2-methylimidazol, N,N-Dimethylaminomethyl(meth)acrylat, N,N-Diethylaminomethyl (meth) acrylat, N,N-Dimethylaminoethyl (meth) acrylat, N,N-Diethylaminoethyl (meth)-acrylat, N,N-Dimethylaminobutyl(meth)acrylat, N,N-Diethylaminobutyl(meth) acrylat, N,N-Dimethylaminohexyl (meth) acrylat, N,N-Dimethylaminooctyl(meth)acrylat, N,N-Dimethylaminododecyl (meth)-acrylat, N-[3-(dimethylamino)propyl]methacrylamid, N-[3-(dimethylamino)propyl]acrylamid, N-[3-(dimethylamino)butyl]methacrylamid, N-[8-(dimethylamino)octyl]methacrylamid,N-[12-(dimethylamino) dodecyl]methacrylamid, N-[3-(diethylamino)propyl]-methacrylamid, N-[3-(diethylamino)propyl]acrylamid;

Maleinsäure, Fumarsäure, Maleinsäureanhydrid und seine Halbester, Crotonsäure, Itaconsäure, Diallyldimethylammoniumchlorid, Vinylether (zum Beispiel: Methyl-, Ethyl-, Butyl-, oder Dodecylvinylether), Vinylformamid, Vinylmethylacetamid, Vinylamin; Methylvinylketon, Maleimid, Vinylpyridin, Vinylimidazol, Vinylfuran, Styrol, Styrolsulfonat, Allylalkohol, und Mischungen daraus.

Von diesen sind besonders bevorzugt Acrylsäure, Methacrylsäure, Maleinsäure, Fumarsäure, Crotonsäure, Maleinsäureanhydrid sowie dessen Halbester, Methylacrylat, Methylmethacrylat, Ethylacrylat, Ethylmethacrylat, n-Butylacrylat, n-Butylmethacrylat, t-Butylacrylat, t-Butylmethacrylat, Isobutylacrylat, Isobutylmethacrylat, 2-Ethylhexylacrylat, N-t-Butylacrylamid, N-Octylacrylamid, 2-Hydroxyethylacrylat, Hydroxypropylacrylat, 2-Hydroxyethylmethacrylat, Hydroxypropylmethacrylat, Alkylenglykol(meth)-acrylate, ungesättigte Sulfonsäuren wie zum Beispiel Acrylamidopropansulfonsäure, Vinylpyrrolidon, Vinylcaprolactam, Vinylether (z.B.: Methyl-, Ethyl-, Butyl-, oder Dodecylvinylether), Vinylformamid, Vinylmethylacetamid, Vinylamin, 1-Vinylimidazol, 1-Vinyl-2-methylimidazol, N,N-Dimethylaminomethylmethacrylat und N-[3-(dimethylamino)propyl]methacrylamid; 3-Methyl-1-vinylimidazoliumchlorid, 3-Methyl-1-vinylimidazoliummethylsulfat, N,N-Dimethylaminoethylmethacrylat, N-[3-(dimethylamino)propyl]-methacrylamid quaternisiert mit Methylchlorid, Methylsulfat oder Diethylsulfat.

Monomere, mit einem basischen Stickstoffatom, können dabei auf folgende Weise quarternisiert werden:

Zur Quaternisierung der Amine eignen sich beispielsweise Alkylhalogenide mit 1 bis 24 C-Atomen in der Alkylgruppe, z.B. Methylchlorid, Methylbromid, Methyliodid, Ethylchlorid, Ethylbromid, Propylchlorid, Hexylchlorid, Dodecylchlorid, Laurylchlorid und Benzylhalogenide, insbesondere Benzylchlorid und Benzylbromid. Weitere geeignete Quaternisierungsmittel sind Dialkylsulfate, insbesondere Dimethylsulfat oder Diethylsulfat. Die Quaternisierung der basischen Amine kann auch mit Alkylenoxiden wie Ethylenoxid oder Propylenoxid in Gegenwart von Säuren durchgeführt werden. Bevorzugte Quaternisierungsmittel sind: Methylchlorid, Dimethylsulfat oder Diethylsulfat.

Die Quaternisierung kann vor der Polymerisation oder nach der Polymerisation durchgeführt werden.

Außerdem können die Umsetzungsprodukte von ungesättigten Säuren, wie z.B. Acrylsäure oder Methacrylsäure, mit einem quaternisierten Epichlorhydrin der allgemeinen Formel (V) eingesetzt werden (R¹⁸ = C₁-C₄₀ Alkyl).

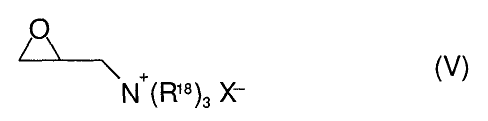

Beispiele hierfür sind zum Beispiel: (Meth)acryloyloxyhydroxypropyltrimethylammoniumchlorid und (Meth) acryloyloxyhydroxypropyltriethylammoniumchlorid.

Die eingesetzten Monomere können, sofern sie ionisierbare Gruppen enthalten, vor oder nach der Polymerisation, zum Teil oder vollständig mit Säuren oder Basen neutralisiert werden, um so z.B. die Wasserlöslichkeit oder -dispergierbarkeit auf ein gewünschtes Maß einzustellen.

Als Neutralisationsmittel für Säuregruppen tragende Monomere, Präpolymere oder Polymere können zum Beispiel Mineralbasen wie Natriumcarbonat, Alkalihydroxide sowie Ammoniak, organische Basen wie Aminoalkohole speziell 2-Amino-2-Methyl-1-Propanol, Monoethanolamin, Diethanolamin, Triethanolamin, Triisopropanolamin, Tri[(2-hydroxy)1-propyl] amin, 2-Amino-2-Methyl-1,3-Propandiol, 2-Amino-2-hydroxymethyl-1,3-Propandiol sowie Diamine wie zum Beispiel Lysin verwendet werden.

Als Neutralisationsmittel für kationisierbare Gruppen tragende Monomere, Präpolymere oder Polymere können zum Beispiel Mineralsäuren wie Salzsäure, Schwefelsäure oder Phosphorsäure, sowie organische Säuren wie Carbonsäuren, Milchsäure, Zitronensäure oder andere eingesetzt werden.

Zusätzlich zu den oben genannten Monomeren können als Monomere sogenannte Makromonomere wie zum Beispiel silikonhaltige Makromonomere mit ein oder mehreren radikalisch polymerisierbaren Gruppen eingesetzt werden. Derartige Makromonomere sind beispielsweise in in EP 0408311 (A2, B1) und EP 0412704 (A2, B1), auf die hier ausdrücklich Bezug genommen wird, beschrieben.

Des weiteren können fluorhaltige Monomere wie sie beispielsweise in der EP 558423 (A1, B1) beschrieben sind, vernetzend wirkende oder das Molekulargewicht regelnde Verbindungen in Kombination oder alleine eingesetzt werden.

Als Regler können die üblichen dem Fachmann bekannten Verbindungen wie zum Beispiel Schwefelverbindungen (z.B.: Mercaptoethanol, 2-Ethylhexylthioglykolat, Thioglykolsäure oder Dodecylmercaptan) sowie Tribromchlormethan oder andere Verbindungen die regelnd auf das Molekulargewicht der erhaltenen Polymerisate wirken, verwendet werden.

Es können gegebenenfalls auch thiolgruppenhaltige Silikon-verbindungen eingesetzt werden.

Als vernetzende Monomere können Verbindungen mit mindestens zwei ethylenisch ungesättigten Doppelbindungen eingesetzt werden wie zum Beispiel Ester von ethylenisch ungesättigten Carbonsäuren, wie Acrylsäure oder Methacrylsäure und mehrwertigen Alkoholen, Ether von mindestens zweiwertigen Alkoholen wie zum Beispiel Vinylether oder Allylether. Außerdem geeignet sind geradkettige oder verzweigte, lineare oder cyclische aliphatische oder aromatische Kohlenwasserstoffe, die über mindestens zwei Doppelbindungen verfügen, welche bei den aliphatischen Kohlenwasserstoffen nicht konjugiert sein dürfen. Ferner geeignet sind Amide der Acryl- und Methacrylsäure und N-Allylamine von mindestens zweiwertigen Aminen wie zum Beispiel (1,2-Diaminoethan, 1,3-Diaminopropan). Ferner sind Triallylamin oder entsprechende Ammoniumsalze, N-Vinylverbindungen von Harnstoffderivaten, mindestens zweiwertigen Amiden, Cyanuraten oder Urethanen. Weitere geeignete Vernetzer sind Divinyldioxan, Tetraallylsilan oder Tetravinylsilan.

Besonders bevorzugte Vernetzer sind beispielsweise Methylenbisacrylamid, Triallylamin und Triallylammoniumsalze, Divinylimidazol, N,N'-Divinylethylenharnstoff, Umsetzungsprodukte mehrwertiger Alkohole mit Acrylsäure oder Methacrylsäure, Methacrylsäureester und Acrylsäureester von Polyalkylenoxiden oder mehrwertigen Alkoholen die mit Ethylenoxid und/oder Propylenoxid und/oder Epichlorhydrin umgesetzt worden sind.

Weitere für das Verfahren vorteilhaft einzusetzende kosmetische Formulierungen enthalten ethylenisch ungesättigte Präpolymere und zusätzlich ggf. einen oder mehrerer Reaktivverdünner.

Als Präpolymere kommen beispielsweise (meth)acrylatfunktionelle (Meth) Acrylcopolymere, Polyether(meth) acrylate, Polyester(meth)acrylate, ungesättigte Polyester, Epoxy(meth)acrylate, Urethan(meth)acrylate, Amino (meth) acrylate, Melamin(meth) acylate, Silikon(meth)acrylate zum Einsatz. Bevorzugt werden Urethan(meth)acrylate, Polyester(meth)acrylate und/oder aliphatische Urethanacrylate eingesetzt. Die Präpolymere weisen üblicherweise ein zahlenmittleres Molakulargewicht von 500 bis 50000, bevorzugt von 500 bis 5000, auf.

Bevorzugt werden Präpolymere eingesetzt, die pro Molekül mindestens 2, besonders bevorzugt 3 bis 6 Doppelbindungen aufweisen. Bevorzugt weisen die verwendeten Bindemittel außerdem ein Doppelbindungsäquivalentgewicht von 400 bis 2000, besonders bevorzugt von 500 bis 900, auf. Außerdem weisen die Bindemittel bevorzugt eine Viskosität von 250 bis 11000 mPas s auf.

Polyester(meth)acrylate sind dem Fachmann prinzipiell bekannt. Sie sind durch verschiedene Methoden herstellbar. Beispielsweise kann Acrylsäure und/oder Methacrylsäure direkt als Säurekomponente beim Aufbau der Polyester eingesetzt werden. Daneben besteht die Möglichkeit, Hydroxyalkylester der (Meth)Acrylsäure als Alkoholkomponente direkt beim Aufbau der Polyester einzusetzen. Bevorzugt werden die Polyester(meth)acrylate aber durch Acrylierung von Polyestern hergestellt. Beispielsweise können zunächst hydroxylgruppenhaltige Polyester aufgebaut werden, die dann mit Acryl- oder Methacrylsäure umgesetzt werden. Es können auch zunächst carboxylgruppenhaltige Polyester aufgebaut werden, die dann mit einem Hydroxyalkylester der Acryl- oder Methacrylsäure umgesetzt werden. Nicht umgesetzte (Meth)Acrylsäure kann durch Auswaschen, Destillieren oder bevorzugt durch Umsetzten mit einer äquivalenten Menge einer Mono- oder Diepoxidverbindung unter Verwendung geeigneter Katalysatoren, wie z.B. Triphenylphosphin, aus dem Reaktionsgemisch entfernt werden.

Polyether(meth)acrylate sind dem Fachmann ebenfalls prinzipiell bekannt. Sie sind durch verschiedene Methoden herstellbar. Beispielsweise können hydroxylgruppenhaltige Polyether, die mit Acrylsäure und/oder Methacrylsäure verestert werden, durch Umsetzung von zwei- und/oder mehrwertigen Alkoholen mit verschiedenen Mengen an Ethylenoxid und/oder Propylenoxid nach gut bekannten Methoden (vgl. z.B. Houben-Weyl, Band XIV, 2, Makromolekulare Stoffe II, (1963)) erhalten werden. Einsetzbar sind auch Polymerisationsprodukte des Tetrahydrofurans oder Butylenoxids.

Eine Flexibilisierung der Polyether(meth)acrylate und der Polyester(meth)acrylate ist beispielsweise dadurch möglich, daß entsprechende OH-funktionelle Präpolymere bzw. Oligomere (Polyether- oder Polyester-Basis) mit längerkettigen, aliphatischen Dicarbonsäuren, insbesondere aliphatischen Dicarbonsäuren mit mindestens 6 C-Atomen, wie beispielsweise Adipinsäure, Sebacinsäure, Dodecandisäure und/oder Dimerfettsäuren, umgesetzt werden. Diese Flexibilisierungsreaktion kann dabei vor oder auch nach der Addition von Acryl- bzw. Methacrylsäure an die Oligomere bzw. Präpolymere durchgeführt werden.

Ferner sind auch Epoxy(meth)acrylate dem Fachmann als Beschichtungsmittel wohl bekannt und brauchen daher nicht näher erläutert zu werden. Sie werden üblicherweise hergestellt durch Anlagerung von Acrylsäure an Epoxidharze, beispielsweise an Epoxidharze auf Basis Bisphenol A oder anderer handelsüblicher Epoxidharze.

Eine Flexibilisierung der Epoxy(meth)acrylate ist beispielsweise analog dadurch möglich, daß entsprechende epoxy-funktionelle Präpolymere bzw. Oligomere mit längerkettigen, aliphatischen Dicabonsäuren, insbesondere aliphatischen Dicarbonsäuren mit mindestens 6 C-Atomen, wie beispielsweise Adipinsäure, Sebacinsäure, Dodecandisäure und/oder Dimerfettsäuren, umgesetzt werden. Diese Flexibilisierungsreaktion kann dabei vor oder nach der Addition von Acryl- bzw. Methacrylsäure an die Oligomere bzw. Präpolymere durchgeführt werden.

Urethan(meth)acrylate sind dem Fachmann ebenfalls wohl bekannt und brauchen daher nicht näher erläutert zu werden. Sie können erhalten werden durch Umsetzung eines Di- oder Polyisocyanates mit einem Kettenverlängerunsmittel aus der Gruppe der Diole/ Polyole und/oder Diamine/Polyamine und/oder Dithiole/Polythiole und/oder Alkanolamine und anschließende Umsetzung der restlichen freien Isocyanatgruppen mit mindestens einem Hydroxyalkyl(meth)acrylat oder Hydroxyalkylester anderer ethylenisch ungesättigter Carbonsäuren.

Die Mengen an Kettenverlängerungsmittel, Di- bzw. Polyisocyanat und Hydroxyalkylester werden dabei bevorzugt so gewählt, daß
1. das Äquivalentverhältnis der NCO-Gruppen zu den reaktiven Gruppen des Kettenverlängerungsmittels (Hydroxyl-, Amino- bzw. Mercaptylgrupppen) zwischen 3:1 und 1:2, bevorzugt bei 2:1, liegt und
2. die OH-Gruppen der Hydroxyalkylester der ethylenisch ungesättigten Carbonsäuren in stöchiometrischen Mengen in bezug auf die noch freien Isocyanatgruppen des Präpolymeren aus Isocyanat und Kettenverlängerungsmittel vorliegen.

Außerdem ist es möglich, die Polyurethanacrylate herzustellen, indem zunächst ein Teil der Isocyanatgruppen eines Di- oder Polyisocyantes mit mindestens einem Hydroxyalkylester umgesetzt wird und die restlichen Isocyanatgruppen anschließend mit einem Kettenverlängerungsmittel umgesetzt werden. Auch in diesem Fall werden die Mengen an Kettenverlängerungsmittel, Isocyanat und Hydroxyalkylester so gewählt, daß das Äquivalentverhältnis der NCO-Gruppen zu den reaktiven Gruppen des Kettenverlängerungsmittels zwischen 3:1 und 1:2, bevorzugt bei 2:1 liegt und das Äquivalentverhältnis der restlichen NCO-Gruppen zu den OH-Gruppen des Hydroxyalkylesters 1 : 1 beträgt. Selbstverständlich sind auch sämtliche Zwischenformen dieser beiden Verfahren möglich. Beispielsweise kann ein Teil der Isocyanatgruppen eines Diisocyanates zunächst mit einem Diol umgesetzt werden, anschließend kann ein weiterer Teil der Isocyanatgruppen mit dem Hydroxalkylester und im Anschluß hieran können die restlichen Isocyanatgruppen mit einem Diamin umgesetzt werden.

Diese verschiedenen Herstellverfahren der Polyurethanacrylate sind bekannt (vgl. z.B. EP-A-203 161) und bedürfen daher keiner genauen Beschreibung.

Eine Flexibilisierung der Urethan(meth)acrylate ist beispielsweise dadurch möglich, daß entsprechende isocyanat-funktionelle Präpolymere mit längerkettigen, aliphatischen Diolen und/oder Diaminen, insbesondere aliphatischen Diolen und/oder Diaminen mit mindestens 6 C-Atomen umgesetzt werden. Diese Flexibilisierungsreaktion kann dabei vor oder nach der Addition von Acryl- bzw.

Methacrylsäure an die Oligomere bzw. Prepolymer durchgeführt werden.

Die Präpolymere können ggf. zusammen mit einem oder mehreren Reaktivverdünnern zum Einsatz kommen. Die Reaktivverdünner können dabei ethylenisch ungesättigte Verbindungen sein. Die Reaktivverdünner können mono-, di- oder polyungesättigt sein. Sie dienen üblicherweise zur Beeinflussung des rheologischen Verhaltens und der Materialeigenschaften.

Der bzw. die Reaktivverdünner werden in den Präpolymeren bevorzugt in einer Menge von 0 bis 70 Gew.-%, besonders bevorzugt von 15 bis 65 Gew.-%, jeweils bezogen auf das Gesamtgewicht des Präpolymers, eingesetzt.

Als Reaktivverdünner werden beispielsweise (Meth)Acrylsäure und deren Ester, Maleinsäure und deren Ester bzw. Halbester, Vinylacetat, Vinylether, Vinylharnstoffe u.ä. eingesetzt. Beispiele sind Alkylenglykoldi(meth) acrylat, Polyethylenglykoldi(meth)acrylat, 1,3-Butandioldi (meth) acrylat, Vinyl (meth) acrylat, Allyl (meth) acrylat, Glycerin-tri (meth)acrylat, Trimethylolpropantri(meth)acrylat, Trimethylolpropandi (meth) acrylat, Styrol, Vinyltoluol, Divinylbenzol, Pentaerythrittri(meth)acrylat, Pentaerythrittetra(meth) acrylat, Dipropylenglykoldi (meth)acrylat, Hexandioldi(meth)acrylat, Ethoxyethoxyethylacrylat, N-Vinylpyrrolidon, Phenoxyethylacrylat, Dimethylaminoethylacrylat, Hydroxyethyl (meth) acrylat, Butoxyethylacrylat, Isobornyl(meth)acrylat, Dimethylacrylamid und Dicyclopentylacrylat, die in der EP-A-250 631 beschriebenen, langkettigen linearen Diacrylate mit einem Molekulargewicht von 400 bis 4000, bevorzugt von 600 bis 2500. Beispielsweise können die beiden Acrylatgruppen durch eine Polyoxibutylenstruktur getrennt sein. Einsetzbar sind außerdem 1,12-Dodecyldiacrylat und das Umsetzungsprodukt von 2 Molen Acrylsäure mit einem Mol eines Dimerfettalkohols, der im allgemeinen 36 C-Atome aufweist. Geeignet sind auch Gemische der genannten Monomeren.

Bevorzugt werden als Reaktivverdünner Mono- und/oder Diacrylate, wie z.B. Isobornylacrylat, Hexandioldiacrylat, Tripropylenglykoldiacrylat und Laromer™ 8887 der Firma BASF Aktiengesellschaft eingesetzt. Besonders bevorzugt werden Isobornylacrylat, Hexandioldiacrylat und Tripropylenglykoldiacrylat eingesetzt.

Weitere für das Verfahren vorteilhaft einzusetzende kosmetische Formulierungen enthalten ungesättigte Verbindungen, die sich von den ungesättigten Fettsäuren ableiten; beispielsweise Triglyceride und Alkydharze (vgl. DIN 53183). Die Alkydharze können beispielsweise als Alkydlacke (vgl. DIN 55945) formuliert werden.

Weiterhin enthalten die erfindungsgemäßen Beschichtungsmittel gegebenenfalls noch übliche Hilfsmittel und/oder Additive, beispielsweise Lichtschutzmittel (z.B. HALS-Verbindungen, Benztriazole, Oxalanilid u.ä.), Slipadditive, Polymerisationsinhibitoren, Mattierungsmittel, Entschäumer, Verlaufsmittel und filmbildende Hilfsmittel, z.B. Cellulose-Derivate, oder andere Additive.

Erfindungsgemäß ist außerdem die Verwendung kosmetischer Formulierungen, die Phenol-, Melamin-, und Formaldehydharze enthalten oder die Stoffe enthalten, aus denen diese Harze unter Einwirkung von NIR-Strahlung entstehen. Insbesondere sind vernetzende Systeme geeignet, wie sie in U.S. 4,588,760 und U.S. 4,278,659 genannt werden und auf die hier ausdrücklich Bezug genommen wird. Weitere besonders geeignete Inhaltsstoffe sind die in DE-A 198 165 27 beschriebenen― Polymere und Dispersionen.

Erfindungsgemäß ist außerdem die Verwendung kosmetischer Formulierungen, die unter Einwirkung von NIR-Strahlung Makromoleküle mit Si-O-Si-Gruppierungen bilden. Hierbei sind insbesondere die in DE-A 198 227 22 genannten Inhaltsstoffe und Formulierungen geeignet, auf die hier ausdrücklich Bezug genommen wird.

Die erfindungsgemäßen kosmetischen Formulierungen können weiterhin Farbstoffe enthalten. Insbesondere sind Farbstoffe, die NIR-Strahlung absorbieren, geeignet. NIR-absorbierende Farbstoffe sind dem Fachmann bekannt und in der Literatur beschrieben. Besonders bevorzugt sind hierbei NIR-Polymerisationsinitiatoren und NIR-Photokatalysatoren, wie sie in EP-A 774 492, U.S. 5,607,814, U.S. 5,686,639, U.S. 5,858,604 und der dort genannten Literatur beschrieben sind und auf die hier ausdrücklich Bezug genommen wird.

Beispielsweise sind kationische Farbstoffe, die wenigstens eine der folgenden Gruppierungen enthalten, 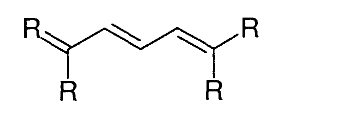 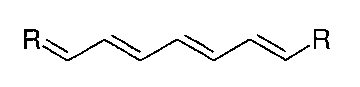 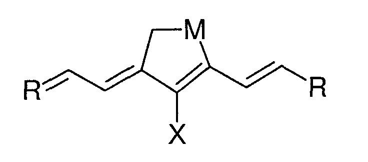 wobei
- R: für gleiche oder ungleiche aromatische oder chinoide Reste steht,
- X: für -Cl oder für -N(Phenyl)₂ steht und
- M: für -CH₂- oder -CH₂-CH₂- steht
geeignet. Ein Beispiel für derartige Verbindungen ist der kationische NIR-Farbstoff (**1**) :

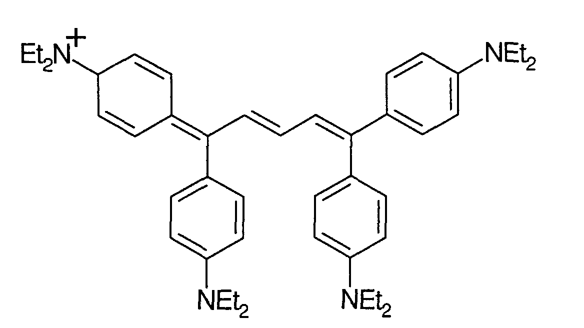

Den oben genannten Monomeren, Präpolymeren und Polymeren können zur Verbesserung der Wirksamkeit der NIR-Strahlung Verbindungen zugesetzt werden, die Gruppierungen R-O-O-R oder R'-N=N-R' enthalten. Dies ist insbesondere von Interesse, wenn die Verbindungen ethylenisch ungesättigte Gruppierungen enthalten. R steht für gleiche oder ungleichartige organische Reste oder für Wasserstoff. R' steht für gleiche oder ungleichartige organische Reste. Bevorzugt sind Wasserstoffperoxid, Hydroperoxide, Peroxide, Percarbonate und Perester. Ferner können anorganische Perverbindungen wie Natrium-, Kalium- und Ammoniumperoxodisulfat zugesetzt werden. Weitere geeignete Initiatoren sind die dem Fachmann bekannten Redox-Initiatorsysteme, z.B. Fe(II)/H₂O₂, Weinsäure/H₂O₂, t-Butylhydroperoxid/Weinsäure, Ascorbinsäure/H₂O₂ oder t-Butylhydroperoxid/Ascorbinsäure.

Geeignete Verbindungen vom Typ R-O-O-R sind beispielsweise Acetylcyclohexansulfonyl-peroxid, Dicetyl-peroxy-dicarbonat, Diisopropyl-peroxydicarbonat, t-Amylperneodecanoat, t-Butylpermeodecanoat, Bis-(2,4-dichlorbenzoyl)-peroxid, t-Butylperpivalat, Bis-(3,5,5-trimethylhexanoyl)-peroxid, Dioctanoylperoxid, Didecanoylperoxid, Dilauroylperoxid, Bis-(2-methylbenzoyl)-peroxid, Succinylperoxid, Diacetylperoxid, Dibenzoylperoxid, t-Butylper-2-ethylhexanoat, Bis-(4-chlorbenzoyl)-peroxid, t-Butylperisobutyrat, t-Butylpermaleinat, 1,1 -Bis-(t-butylperoxy)-3,5,5-trimethyl-cyclohexan, 1,1 -Bis-(t-butylperoxy)-cyclohexan, t-Butylperoxy-isopropylcarbonat, t-Butyl-per-3,5,5-trimethylhexanoat, 2,5-Dimethyihexan-2,5-diperbenzoat, t-Butylperacetat, t-Butylperbenzoat, 2,2-Bis-(t-butylperoxy)-butan, 2,2-Bis-(t-butylperoxy)-propan, Dicumylperoxid, 2,5-Dimethylhexan-2,5-di-t-butylperoxid, 3-t-Butylperoxy-3-phenylphthalid, Di-t-amylperoxid, α-α'-Bis-(t-butylperoxy-isopropyl)-benzol, 3,5-Bis (t-butylperoxy)-3,5-dimethyldioxolan-1,2, Di-t-butylperoxid 2,5-Dimethyl-hexin-3-2,5-di-t-butylperoxid, 3,3,6,6,9,9-Hexamethyl-1,2,4,5-tetraoxa-cyclononan, p-Methanhydroperoxid, Pinanhydroperoxid, Diisopropylbenzol-mono-α-hydroperoxid, Cumolhydroperoxid, t-Butylhydroperoxid, Dimyristyl-peroxydicarbonat, Dicyclohexyl-peroxydicarbonat, Bis-(4-t-butylcyclohexyl)-peroxydicarbonat, Di-n-butyl-peroxydicarbonat, Di-2-ethylhexyl-peroxydicarbonat, Diisotridecyl-peroxydicarbonat.

Geeignete Verbindungen vom Typ R'-N=N-R' sind beispielsweise 2,2'-Azobis(4-methoxy-2,4-dimethylvaleronitnl), 2,2'-Azobis(2,4-dimethylvaleronitril), 2,2'-Azobis(isobutyronitril), Dimethyl-2,2'-azobis(isobutyrat), 2,2'-Azobis(2-methyl-butyronitril), 1,1'-Azobis(1-cyclo-hexancarbonitril), 2-(Carbamoylazo)-isobutyronitril, 2,2'-Azobis(2,4,4-trimethyl-pentan), 2,2'-Azobis(N,N' dimethyleneisobutyramidin)dihydrochlorid, 2,2'-Azobis(2-amidino-propan)dihydrochlorid, 2,2'-Azobis(N,N' dimethylenisobutyramidin), 4,4'-Azobis(4-cyano-pentansäure), 2,2'-Azobis [2-methyl-N-(2-hydroxyethyl)propionsäureamid].

Anstelle von Verbindungen des Typs R-O-O-R und R'-N=N-R' sind auch Verbindungen R-R geeignet, die unter für die Anwendung typischen Bedingungen in Radikale zerfallen, beispielsweise 3,4-Dimethyl-3,4-diphenylhexan und 3,4-Dimethyl-3,4-diphenylbutan.

Den oben genannten Monomeren, Präpolymeren und Polymeren können zur Verbesserung der Wirksamkeit der NIR-Strahlung die dem Fachmann bekannten Sikkative zugesetzt werden. Das sind Trocknungsmittel, insbesondere für ungesättigte Verbindungen und ihre Derivate (z.B. Alkydharze, Triglyceride). Ein Beispiel dafür ist Mangan(II)acetat. Besonders geeignet sind Sikkative, deren Wirkung durch NIR-Strahlung verstärkt wird.

Die folgenden Zusätze werden insbesondere dann in den kosmetischen Mitteln für das erfindungsgemäße Verfahren eingesetzt, wenn die oben genannten Monomere, Präpolymere und Polymere Epoxid-Gruppierungen enthalten:

Aliphatische Polyamine H₂N(CH₂CH₂NH)ₙH, z.B Diethylentriamine (DETA, n = 2) und Triethylentetramine (TETA, n = 3), aliphatische Diamine auf Basis von Propylenoxid und Ammoniak H₂N(CH₂CH(CH₃)O)ₙCH₂CH(CH₃)NH₂, z.B. Jeffamine D-230 (Texaco Chemical Co.) mit n = 2 bis 3, Polyamiddiamine oder Amidopolyamine (z.B. aus der Reaktion von Dimerfettsäuren mit DETA oder TETA), cycloaliphatische Diamine, z.B. Cyclohexan-1,2-diamin, 4-(2-Aminopropane-2-yl)-1-methylcyclohexan-1-amin, oder Menthandiamin, aromatische Diamine, z.B. Bis(4-aminophenyl)methan (MDA or methylene dianiline) und Bis(4-aminophenyl)sulfone (DADS, DDS, or dapsone), Carbonsäureanhydride, z.B. Methylbicyclo[2.2.1]hepten-2,3-dicarbonsäureanhydrid, 1,2-Cyclohexanedicarbonsäureanhydrid, Hexahydrophthalsäureanhydrid oder Phthalsäureanhydrid, tertiäre Amine NR₃, wobei gleiche oder ungleichartige organische Reste (bevozugt Alkylreste) sind, Melamin-, Harnstoff- und Phenolformaldehyd-Addukte (auch Aminoplaste, Phenoplaste), Polycarbonsäurepolyester, Dicyandiamid, Lewis-Säuren und -Basen, z.B. Bortrifluorid komplexiert mit Methylethylamin, 2,4,6-Tris(N,N-dimethylaminomethyl)phenol (Rohm & Haas DMP-30), N,N-Dimethylbenzylamine.

Die oben genannten Polymere, Präpolymere und Monomere sind in dem erfindungsgemäß zu verwendenden kosmetischen Mittel zu 0,05 bis 99 Gew.-%, bevorzugt 0,1 bis 50 Gew.-% und besonders bevorzugt zu 0,2 bis 20 Gew.-% enthalten. In einer besonders bevorzugten Ausführung enthalten die kosmetischen Mittel zusätzlich mehr als 50 Gew.-% Fluide, die beispielsweise als Lösungsmittel oder Treibmittel wirken können. Beispiele für diese Fluide sind Wasser, Ethanol, Isopropanol, Propan, Butan, Dimethylether, Diethylether, Kohlendioxid, Druckluft, Hexafluorethan und Decamethylcyclopentasiloxan.

Die erfindungsgemäß eingesetzten kosmetischen Formulierungen können neben den oben genannten Stoffen und ihren Mischungen in der Kosmetik übliche Hilfsstoffe enthalten, wie Weichmacher, Filmbildehilfsmittel, Pigmente, Parfums, Verdicker, Tenside, Konservierungsmittel, kosmetische Wirkstoffe wie Phytantriol, Vitamine und Provitamine, beispielsweise Vitamin A, E und C, Retinol, Bisabolol, Panthenol, natürliche und synthetische Lichtschutzmittel Naturstoffe, Treibungsmittel, Lösungsvermittler, Repellents, Bleichmittel, Färbemittel, Tönungsmittel, Bräununsmittel, Reflektoren, Proteine, Ceramid, AH-Säuren (alpha-Hydroxycarbonsären wie z.B. Milchsäure und Salicylsäure) sowie deren Salze, Fruchtsäuren, Collagen, Eiweißhydrolysate, Stabilisatoren, pH-Wert Regulatoren, Emulgatoren, Gelbildner, Konsistenzgeber, Silikone, Feuchthaltemittel, Rückfetter, UV-Schutzmittel oder andere übliche Additive, allein oder in Kombination zugefügt werden.

Die Hilfsstoffe können bei der Polymerisation anwesend sein und/oder nach der Polymerisation zugefügt werden.

Als weitere übliche Zusätze können enthalten sein Fettkörper, wie mineralische und synthetische Öle, wie z.B. Paraffine, Siliconöle und aliphatische Kohlenwasserstoffe mit mehr als 8 Kohlenstoffatomen, tierische und pflanzliche Öle, wie z.B. Sonnenblumenöl, kokosöl, Avocadoöl, Olivenöl, Lanolin, oder wachse, Fettsäuren, Fettsäureester, wie z.B. Triglyceride von C₆-C₃₀-Fettsäuren, Wachsester, wie z.B. Jojobaöl, Fettalkohole, Vaseline, hydriertes Lanolin. Selbstverständlich können auch Mischungen derselben verwendet werden.

Übliche Verdickungsmittel in derartigen Formulierungen sind vernetzte Polyacrylsäuren und deren Derivate, Polysaccharide wie Xanthan-gum, Agar-Agar, Alginate oder Tylosen, Cellulosederivate, z.B. Carboxymethylcellulose oder Hydroxycarboxymethylcellulose, Fettalkohole, Monoglyceride und Fettsäuren, Polyvinylalkohol und Polyvinylpyrrolidon.

Weiterhin können auch keimhemmende Mittel eingesetzt werden. Dazu gehören generell alle geeigneten Konservierungsmittel mit spezifischer Wirkung gegen grampositive Bakterien, z.B. Triclosan (2,4,4'-Trichlor-2'-hydroxydiphenylether), Chlorhexidin (1,1'-Hexamethylenbis [5-(4-chlorphenyl)-biguanid) sowie TTC (3,4,4'-Trichlorcarbanilid). Quartäre Ammonium-Verbindungen sind prinzipiell ebenfalls geeignet, werden jedoch bevorzugt für desinfizierende Seifen und Waschlotionen verwendet. Auch zahlreiche Riechstoffe haben antimikrobielle Eigenschaften. Spezielle Kombinationen mit besonderer Wirksamkeit gegenüber grampositiven Bakterien werden für die Komposition sog. Deoparfums eingesetzt. Auch eine große Anzahl etherischer Öle bzw. deren charakteristische Inhaltsstoffe wie z.B. Nelkenöl (Eugenol), Minzöl (Menthol) oder Thymianöl (Thymol), zeigen eine ausgeprägte antimikrobielle Wirksamkeit. Die antibakteriell wirksamen Stoffe werden bevorzugt in Konzentrationen von ca. 0,1 bis 0,3 Gew.-% eingesetzt.

Als geeignete Lösungsmittel sind insbesondere zu nennen Wasser und niedrige Monoalkohol oder Polyole mit 1 bis 6 Kohlenstoffatomen und Mischungen davon; bevorzugte Monoalkohole oder Polyole sind Ethanol, i-Propanol, Propylenglycol, Glycerin und Sorbit. Weitere bevorzugte Hilfsstoffe sind Lösemittel und Treibgase aus der Gruppe Dimethylether, Propan, Butan, fluorierte Kohlenwasserstoffe, cyclische Silkone, verzweigte und unverzweigte Alkane, organische Ester und Ether.

Die erfindungsgemäß eingesetzten kosmetischen Formulierungen können neben den oben genannten Stoffen und ihren Mischungen auch zusätzliche Polymere wie zum Beispiel Polyamide, Polyurethane, Polyester, Homo- und Copolymere von ethylenisch ungesättigten Monomeren zugegen sein. Bevorzugte Homo- und Copolymere sind aus den oben genannten Monomeren erhältlich. Diese können durch Carboxylatgruppen, Sulfonatgruppen, Phosphonatgruppen sowie durch stickstoffhaltige kationische Gruppierungen funktionalisiert sein.

Ferner können Silikone und alkoxylierte Silikone und davon abgeleitete Verbindungen zugegen sein. Beispiele für solche zum Teil auch in der Kosmetik eingesetzten Polymeren sind in der folgenden Liste aufgeführt:

| | | |
|---|---|---|
| INCI/CTFA-Bezeichnung | Polymer | Hersteller |
| Acrylat-Copolymer | Amerhold | Amerchol |
| PVP / Acrylat-Copolymer | Luviflex VBM 35 | BASF |
| PVP / VA | Luviskol VA | BASF |
| Polyvinylcaprolactam | Luviskol Plus | BASF |
| VA / Crotonat Copolymer | Luviset CA 66 | BASF |
| VA / Crotonat/Vinylpropionat Copolymer | Luviset CAP | BASF |
| Acrylat / Acrylamid Copolymer | Ultrahold 8 | BASF |
| Acrylat / Acrylamid Copolymer | Ultrahold Strong | BASF |
| Acrylat Copolymer | Luvimer MAE | BASF |
| Acrylat Copolymer | Luvimer 100P, 36D, 30E | BASF |
| Polyquatemium 46 | Luviquat Hold | BASF |
| Polyurethan-1 | Luviset P.U.R. | BASF |
| Methacryloyl Ethylbetain/Acrylat Copolymer | Diaformer | Clariant |
| Diglycol / CHDM / Isophthalat/SIP Copolymer | Eastman AQ Polymer | Eastman |
| Acrylat/Diacetonacrylamid Copolymer | Plascise L53 | Goo Chemicals |
| Butylacrylat-Lauryl Methacrylat | Diahold EX-55/Plascise L 1210 | Goo Chemicals |
| PVP | PVP K | ISP |
| PVP/VA | PVP/PA | ISP |
| Vinyl Caprolactam / PVP / Dimethylamino-methyl Methacrylat Copolymer | Copolymer VC 713 (= Advantage HC) | ISP |
| Vinyl Caprolactam / PVP / Dimethylamino-methyl Methacrylat Copolymer | H2OLD EP-1 | ISP |
| PVM / MA Butylester Copolymer | Gantrez ES 425 | ISP |
| VA / Butylmaleat/Isobornyl Acrylat Copolymer | Advantage Plus (CP, V) | ISP |
| PVM / MA Ethylester Copolymer | Omnirez 2000 | ISP |
| PVP / DMAPA Acrylat Copolymer | Styleeze CC―10 | ISP |
| PVP / Vinylcaprolactam/DMAPA Acrylat Copolymer | Aquaflex SF―40 | ISP |
| N―Methylacryloyl oxethyl N,N―dimethylammonium―a―N―methyl carboxybetain alkyl methacrylat | Yukaformer R205 | Mitsubishi |
| N―Methylacryloyl oxethyl N,N―dimethylammonium―a―N―methyl carboxybetain alkyl methacrylat | Yukaformer SM | Mitsubishi |
| VA/Crotonat / Copolymer | Resyn 28―1310 | National Starch |
| VA/Crotonat / Neodecanoat Copolymer | Resyn 28―2930 | National Starch |
| Octylacrylamid / Acrylat/ Butylaminoethyl Methacrylat Copolymer | Amphomer 28―4910 | National Starch |
| Octylacrylamid/Acrylat/ Butylaminoethyl Methacrylat Copolymer | Amphomer LV―71 | National Starch |
| Acrylat/Octylacrylamid Copolymer | Amphomer HC 28―4942 (= Versatyl) | National Starch |
| Octylacrylamid / Acrylat Copolymer | Versatyl 90 | National Starch |
| Acrylat Copolymer | Balance 0/55 | National Starch |
| Octylacrylamid / Acrylat/Butylaminoethyl Methacrylat Copolymer | Balance 47 (= Lovocryl 47) | National Starch |
| /Acrylat/Hydroxyester Acrylat Copolymer | Acudyne | Rohm & Haas |
| Diglycol/CHDM / Isophthalates / SIP Copolymer | Eastman AQ | Eastman |
| Polyurethane―1 | Luviset P.U.R | BASF |
| PEG / PPG - 25/25 Dimethicone / Acrylates Copolymer | Luviflex Silk | BASF |

Die oben genannten Polyamide sind in EP 0696607 (A1) und in EP 0787480 (A1) beschrieben.

Weitere geeignete kationische Polymere: Mit der Bezeichnung Polyquaternium nach INCI, z.B. Copolymere aus Vinylpyrrolidon/ N-Vinylimidazoliumsalzen (Luviquat™ FC, Luviquat™ HM, Luviquat™ MS, Luviquat™ Care), Copolymere aus N-Vinylpyrrolidon/ Dimethylaminoethylmethacrylat, quaternisiert mit Diethylsulfat (Luviquat™ PQ 11), Copolymere aus N-Vinylcaprolactam/N-Vinylpyrrolidon/N-Vinylimidazoliumsalzen (Luviquat™ Hold); kationische Cellulosederivate (Polyquaternium-4 und -10), Acrylamidcopolymere (Polyquaternium-), Styleeze™ CC-10, Aquaflex™ SF-40, Chitosanderivate sowie Polylysin und Lysincopolymere.

Weitere geeignete betaine Polymere: Beispielsweise Yukaformer™ (R205, SM) und Diaformer™.

Weitere geeignete Copolymere aus N-Vinylpyrrolidon und Vinylpropionat, Polysiloxane, Polyethylenimine und deren Salze, Polyvinylamine und deren Salze, Cellulosederivate, Polyasparaginsäuresalze und Derivate.

Außerdem geeignet sind auch Biopolymere, d.h. Polymere und Monomere, die aus natürlich nachwachsenden Rohstoffen gewonnen werden und aus natürlichen Monomerbausteinen aufgebaut sind, z.B. Cellulosederivate, Chitin-, Chitosan-, DNA-, Hyaloronsäure- und RNA-Derivate, Alkydharze- und ungesättigte Triglyceride.

Die Herstellung der kosmetischen oder dermatologischen Zubereitungen erfolgt nach den üblichen dem Fachmann geläufigen Regeln.

Solche Formulierungen liegen vorteilhafterweise in Form von Emulsionen bevorzugt als Wasser-in-Öl-(W/O)- oder Öl-in-Wasser-(O/W)-Emulsionen vor. Es ist aber auch erfindungsgemäß möglich und gegebenenfalls vorteilhaft andere Formulierungsarten zu wählen, beispielsweise Hydrodispersionen, Gele, Öle, Oleogele, multiple Emulsionen, beispielsweise in Form von W/O/W- oder O/W/O-Emulsionen, wasserfreie Salben bzw. Salbengrundlagen usw.

Die Herstellung erfindungsgemäß brauchbarer Emulsionen erfolgt nach bekannten Methoden.

Die Emulsionen enthalten neben dem erfindungsgemäßen Copolymer übliche Bestandteile, wie Fettalkohole, Fettsäureester und insbesondere Fettsäuretriglyceride, Fettsäuren, Lanolin und Derivate davon, natürliche oder synthetische Öle oder Wachse und Emulgatoren in Anwesenheit von Wasser.

Die Auswahl der Emulsionstyp-spezifischen Zusätze und die Herstellung geeigneter Emulsionen ist beispielsweise beschrieben in Schrader, Grundlagen und Rezepturen der Kosmetika, Hüthig Buch Verlag, Heidelberg, 2. Auflage, 1989, Dritter Teil, worauf hiermit ausdrücklich Bezug genommen wird.

So kann eine erfindungsgemäß brauchbare Hautcreme z.B. als W/O-Emulsion vorliegen. Eine derartige Emulsion enthält eine wäßrige Phase, die mittels eines geeigneten Emulgatorsystems in einer Öl- oder Fettphase emulgiert ist.

Die Konzentration des Emulgatorsystems beträgt in diesem Emulsions-Typ etwa 4 und 35 Gew.-%, bezogen auf das Gesamtgewicht der Emulsion; die Fettphase macht etwa 20 und 60 Gew.-% aus und die wäßrige Phasen etwa 20 und 70 Gew.-%, jeweils bezogen auf das Gesamtgewicht der Emulsion. Bei den Emulgatoren handelt es sich um diejenigen, welche in diesem Emulsionstyp üblicherweise verwendet werden. Sie werden z.B. ausgewählt unter: C₁₂-C₁₈-Sorbitan-Fettsäureestern; Estern von Hydroxystearinsäure und C₁₂-C₃₀-Fettalkoholen; Mono- und Diestern von C₁₂-C₁₈-Fettsäuren und Glyzerin oder Polyglyzerin; Kondensaten von Ethylenoxid und Propylenglycolen; oxypropylenierten/oxyethylenierten C₁₂-C₂₀-Fettalkoholen; polycyclischen Alkoholen, wie Sterolen; aliphatischen Alkoholen mit einem hohen Molekulargewicht, wie Lanolin; Mischungen von oxypropylenierten/polyglycerinierten Alkoholen und Magnesiumisostearat; Succinestern von polyoxyethylenierten oder polyoxypropylenierten Fettalkoholen; und Mischungen von Magnesium-, Calcium-, Lithium-, Zink- oder Aluminiumlanolat und hydriertem Lanolin oder Lanolin-alkohol.

Zu geeigneten Fettkomponenten, welche in der Fettphase der Emulsionen enthalten sein können, zählen Kohlenwasserstofföle, wie Paraffinöl, Purcellinöl, Perhydrosqualen und Lösungen mikrokristalliner Wachse in diesen Ölen; tierische oder pflanzliche Öle, wie Süßmandelöl, Avocadoöl, Calophylumöl, Lanolin und Derivate davon, Ricinusöl, Sesamöl, Olivenöl, Jojobaöl, Karite-Öl, Hoplostethus-Öl; mineralische Öle, deren Destillationsbeginn unter Atmosphärendruck bei ca. 250°C und deren Destillationsendpunkt bei 410°C liegt, wie z.B. Vaselinöl; Ester gesättigter oder ungesättigter Fettsäuren, wie Alkylmyristate, z.B. i-Propyl-, Butyl- oder Cetylmyristat, Hexadecylstearat, Ethyl- oder i-Propylpalmitat, Octan- oder Decansäuretriglyceride und Cetylricinoleat.

Die Fettphase kann auch in anderen Ölen lösliche Siliconöle, wie Dimethylpolysiloxan, Methylphenylpolysiloxan und das Silicon-glycol-Copolymer, Fettsäuren und Fettalkohole enthalten.

Um die Retention von Ölen zu begünstigen, kann man auch Wachse verwenden, wie z.B. Carnauba-Wachs, Candellilawachs, Bienenwachs, mikrokristallines Wachs, Ozokeritwadhs und Ca-, Mg- und Al- Oleate, -Myristate, -Linoleate und -Stearate.

Im allgemeinen werden diese Wasser-in-Öl-Emulsionen so hergestellt, daß die Fettphase und der Emulgator in den Ansatzbehälter gegeben werden. Man erwärmt diesen bei einer Temperatur von 70 bis 75°C, gibt dann die in Öl löslichen Ingredienzien zu und fügt unter Rühren Wasser hinzu, welches vorher auf die gleiche Temperatur erwärmt wurde und worin man die wasserlöslichen Ingredienzien vorher gelöst hat; man rührt, bis man eine Emulsion der gewünschten Feinheit hat, läßt sie dann auf Raumtemperatur abkühlen, wobei gegebenenfalls weniger gerührt wird.

Weiterhin kann eine erfindungsgemäße Pflegeemulsion als O/W-Emulsion vorliegen. Eine derartige Emulsion enthält üblicherweise eine Ölphase, Emulgatoren, die die Ölphase in der Wasserphase stabilisieren, und eine wäßrige Phase, die üblicherweise verdickt vorliegt.

Die wäßrige Phase der O/W-Emulsion der erfindungsgemäßen Zubereitungen enthält gegebenenfalls
- Alkohole, Diole oder Polyole sowie deren Ether, vorzugsweise Ethanol, Isopropanol, Propylenglycol, Glycerin, Ethylenglycolmonoethylether;
- übliche Verdickungsmittel bzw. Gelbildner, wie z.B. vernetzte Polyacrylsäuren und deren Derivate, Polysaccharide wie Xan-than Gum oder Alginate, Carboxymethylcellulose oder Hydroxycarboxymethylcellulose, Fettalkohole, Polyvinylalkohol und Polyvinylpyrrolidon.

Die Ölphase enthält in der Kosmetik übliche Ölkomponenten, wie beispielsweise:
- Ester aus gesättigten und/oder ungesättigten, verzweigten und/oder unverzweigten C₃-C₃₀-Alkancarbonsäuren und gesättigten und/oder ungesättigten, verzweigten und/oder unverzweigten C₃-C₃₀-Alkoholen, aus aromatischen Carbonsäuren und gesättigten und/oder ungesättigten, verzweigten und/oder unverzweigten C₃-C₃₀-Alkoholen, beispielhaft Isopropylmyristat, Isopropylstearat, Hexyldecylstearat, Oleyloleat; außerdem synthetische, halbsynthetische und natürliche Gemische solcher Ester, wie Jojobaöl;
- verzweigte und/oder unverzweigte Kohlenwasserstoffe und -wachse;
- Silikonöle wie Cyclomethicon, Dimethylpolysiloxan, Diethylpolysiloxan, Octamethylcyclotetrasiloxan sowie Mischungen daraus;
- Dialkylether;
- Mineralöle und Mineralwachse;
- Triglyceride gesättigter und/oder ungesättigter, verzweigter und/oder unverzweigter C₈-C₂₄-Alkancarbonsäuren; sie können ausgewählt werden aus synthetischen, halbsynthetischen oder natürlichen Ölen, wie Olivenöl, Palmöl, Mandelöl oder Mischungen.

Als Emulgatoren kommen vorzugsweise O/W-Emulgatoren, wie Polyglycerinester, Sorbitanester oder teilveresterte Glyceride, in Betracht.

Die Herstellung kann durch Aufschmelzen der Ölphase bei ca. 80°C erfolgen; die wasserlöslichen Bestandteile werden in heißem Wasser gelöst, langsam und unter Rühren zur Ölphase zugegeben; homogenisiert und kaltgerührt.

Das erfindungsgemäße Verfahren eignet sich auch zur Verwendung in Wasch- und Duschgel-Formulierungen sowie in Badepräparaten.

Solche Formulierungen enthalten neben den aufgeführten Polymeren üblicherweise anionische Tenside als Basistenside und amphotere und nichtionische Tenside als Cotenside, sowie Lipide, Parfümöle, Farbstoffe, organische Säuren, Konservierungsstoffe und Antioxidantien sowie Verdicker/Gelbildner, Hautkonditioniermittel und Feuchthaltemittel.

In den Wasch, Dusch- und Badepräparaten können alle in Körperreinigungsmitteln üblicherweise eingesetzte anionische, neutrale, amphotere oder kationische Tenside verwendet werden.

Die Formulierungen enthalten 2 bis 50 Gew.-% Tenside, bevorzugt 5 bis 40 Gew.-%, besonders bevorzugt 8 bis 30 Gew-%.

Geeignete anionische Tenside sind beispielsweise Alkylsulfate, Alkylethersulfate, Alkylsulfonate, Alkylarylsulfonate, Alkylsuccinate, Alkylsulfosuccinate, N-Alkoylsarkosinate, Acyltaurate, Acylisethionate, Alkylphosphate, Alkyletherphosphate, Alkylethercarboxylate, Alpha-Olefinsulfonate, insbesondere die Alkali- und Erdalkalimetallsalze, z.B. Natrium, Kalium, Magnesium, Calcium, sowie Ammonium- und Triethanolamin-Salze. Die Alkylethersulfate, Alkyletherphosphate und Alkylethercarboxylate können zwischen 1 bis 10 Ethylenoxid oder Propylenoxid-Einheiten, bevorzugt 1 bis 3 Ethylenoxideinheiten im Molekül aufweisen.

Geeignet sind zum Beispiel Natriumlaurylsulfat, Ammoniumlaurylsulfat, Natriumlaurylethersulfat, Ammoniumlaurylethersulfat, Natriumlaurylsarkosinat, Natriumoleylsuccinat, Ammoniumlaurylsulfosuccinat, Natriumdodecylbenzolsulfonat, Triethanolamindodecylbenzolsulfonat.

Geeignete amphotere Tenside sind zum Beispiel Alkylbetaine, Alkylamidopropylbetaine, Alkylsulfobetaine, Alkylglycinate, Alkylcarboxyglycinate, Alkylamphoacetate- oder -propionate, Alkylamphodiacetate, oder -dipropionate.

Beispielsweise können Cocodimethylsulfopropylbetain, Laurylbetain, Cocamidopropylbetain oder Natriumcocamphopropionat eingesetzt werden.

Als nichtionische Tenside sind beispielsweise geeignet die Umsetzungsprodukte von aliphatischen Alkoholen oder Alkylphenolen mit 6 bis 20 C-Atomen in der Alkylkette, die linear oder verzweigt sein kann, mit Ethylenoxid und/oder Propylenoxid. Die Menge Alkylenoxid beträgt ca. 6 bis 60 Mole auf ein Mol Alkohol. Ferner sind Alkylaminoxide, Mono- oder Dialkylalkanolamide, Fettsäureester von Polyethylenglykolen, ethoxylierte Fettsäureamide, Alkylpolyglykoside oder Sorbitanetherester geeignet.

Außerdem können die Wasch-, Dusch- und Badepräparate übliche kationische Tenside enthalten, wie z.B. quaternäre Ammonium-verbindungen, beispielsweise Cetyltrimethylammoniumchlorid.

Zusätzlich können auch weitere übliche kationische Polymere eingesetzt werden, so z.B. Copolymere aus Acrylamid und Dimethyldiallylammoniumchlorid (Polyquaternium-7), kationische Cellulosederivate (Polyquaternium-4, -10), Guar-hydroxypropyltrimethylammoniumchlorid (INCI: Hydroxypropyl Guar Hydroxypropyltrimonium Chloride), Copolymere aus N-Vinylpyrrolidon und quaternisiertem N-Vinylimidazol (Polyquaternium-16, -44, -46), Copolymere aus N-Vinypyrrolidon/Dimethylaminoethyl-methacrylat, quaternisiert mit Diethylsulfat (Polyquaternium-11) und andere.

Weiterhin können die Wasch- und Duschgel-Formulierungen und Badepräparate Verdicker, wie z.B. Kochsalz, PEG-55, Propylene Glycol Oleate, PEG-120 Methyl Glucose Dioleate und andere, sowie Konservierungsmittel, weitere Wirk- und Hilfsstoffe und Wasser enthalten.

Bei der erfindungsgemäßen Verwendung in der Haarkosmetik, speziell bei der Verwendung als Festiger, ist es vorteilhaft Formulierungen zu verwenden, aus denen nach Applikation eine Glastemperatur größer 20°C resultiert.

### Beispiele

Die unten aufgeführten Formulierungen werden
1. in üblicher Weise auf Haartressen appliziert und aus 25 cm Entfernung für 10 min mit einer NIR-Strahlungsquelle (NIR-Anlage Typ MPP-120-10 der Firma Industrie Servis, D-83052 Bruckmühl) bestrahlt. Die Intensität der NIR-Strahlungsquelle wird so moduliert, daß keine Überhitzung an der Substratoberfläche stattfindet. Alle Haartressen zeigen nach Bestrahlung hohe Formstabilität. Die erfindungsgemäß behandelten Haartressen sind insbesondere nach Besprühen mit Wasser formstabiler als solche, die mit konventionellen Haarfestigern ohne NIR-Strahlung behandelt werden. Beim Bestrahlen mit NIR-Strahlung erwärmen sich die Haartressen weniger stark als bei der Bestrahlung mit einer konventionellen IR-Strahlungsquelle.
2. in für Nagellacke üblicher Weise auf den Fingernagel von Probanden appliziert und wie oben bestrahlt. Es bilden sich Filme mit gegenüber konventionell getrocknetem Nagellack verbesserter Kratzfestigkeit.
3. auf Kollagenfilm, der als Modell für menschliche Haut dient, aufgebracht und wie oben bestrahlt. Der Kollagenfilm ist 24 h in Wasser bei 23°C vorgequollen. Der gravimetrische Vergleich von unbehandeltem und behandeltem Film zeigt, daß der behandelte Film weniger Feuchtigkeit abgibt, als der unbehandelte Film.

### Beispiel 1

### Haarfestigüngsmittel

| | |
|---|---|
| 1,50 g | Polyurethanacrylat (Laromer LR 8987, Fa. BASF, Ludwigshafen/Rh.) |
| 1,50 g | Vinylpyrrolidon/Vinylacetat Copolymer (Luviskol VA 64, |
| | Fa. BASF, Ludwigshafen/Rh.) |
| 0,20 g | 1,2-Propylenglykol |
| 0,15 g | Parfüm |
| 0,03 g | Cetyltrimethylammoniumchlorid |
| 0,008 g | Cumylperoxyneodecanoat (als wäßr. Emulsion, Trigonox |
| | 99-W40, Fa. Akzo Nobel Chemicals, Amersfoort, NL) |
| 20,21 g | Wasser |
| 76,41 g | Ethanol |

### Beispiel 2

### Farbiges Haarfestigungsmittel

| | |
|---|---|
| 0,88 g | Oligoetheracrylat (LR 8863, Fa. BASF, Ludwigshafen/Rh.) |
| 2,63 g | Vinylpyrrolidon/Vinylacetet Copolymer (Luviskol VA 73, |
| | Fa. BASF, Ludwigshafen/Rh.) |
| 0,20 g | 1,2-Propylenglykol |
| 0,15 g | Parfüm |
| 0,05 g | Cetyltrimethylammoniumchlorid |
| 0,005 g | 2,2'-Azobis(2,4,4-trimethyl-pentan), (VR-110 der |
| | Fa. Wako, Osaka, JP) |
| 0,05 g | Basic Brown 17 (C. I. 12 251) |
| 0,01 g | Basic Blue 7 (C. I. 42 595) |
| 0,002 g | Basic Violett 14 (C. I. 42 510) |
| 59,89 g | Wasser |
| 46,28 g | Ethanol |

### Beispiel 3

### 80 % VOC-Pumpspray

| | |
|---|---|
| 1,50 g | anorganisch-organisches Hybridprepolysiloxan aus |
| | Mercaptopropyltrieth-oxysilan, Vinyltriethoxysilan und |
| | wäßrige Salzsäure (1 N) (nach DE 19822722 A1, System 11.) |
| 1,50 g | anorganisch-organisches Hybridprepolysiloxan aus |
| | 3-Glycidoxypropyl-tri-methoxysilan, Tetramethoxysilan, |
| | Tributoxyaluminium, Tetrapropoxyzirko-nium und |
| | Triethanolamin (nach DE 19822722 A1, System 3.) |
| 5,00 g | Vinylacetat/Crotonsäure/Polyethylenoxid Copolymer |
| 0,30 g | Parfüm |
| 11,70 g | Wasser |
| 80,00 g | Ethanol |

### Beispiel 4

### Haarfestigungsmittel mit UV-Schutz

| | |
|---|---|
| 1,00 g | Polyesteracrylat (Laromer LR 8895, Fa. BASF, Ludwigs- |
| | hafen/Rh. |
| 2,00 g | Polyvinylpyrrolidon (Luviskol, Fa. BASF, Ludwigs- |
| | hafen/Rh.) |
| 0,20 g | Parfüm |
| 0,15 g | Glycerin (85prozentig) |
| 0,10 g | 2-Hydroxy-4-methoxybenzophenon |
| 0,01 g | 2,2'-Azobis [2-methyl-N-(2-hydroxyethyl)propionsäureamid] |
| | (VA-086 der Fa. Wako, Osaka, JP) |
| 61,30 g | Wasser |
| 35,25 g | Ethanol |

### Beispiel 5

### Haarfestigungsmittel

| | |
|---|---|
| 1,12 g | Glycerinaldehyd-Resorcin-Vorkondensat nach |
| | U.S. 4,278,659, Formulierung 1 |
| 1,00 g | Lackleinöl |
| 2,12 g | Polyvinylpyrrolidon (Luviskol, Fa. BASF, Ludwigs- |
| | hafen/Rh.) |
| 0,40 g | Hydriertes Rizinusöl, ethoxyliert mit 40 Mol Ethylenoxid |
| 0,10 g | tert-Butylhydroperoxid (in Form der 70% wäßrigen Lösung, |
| | Trigonox A-W70, Fa. Akzo Nobel Chemicals, Amersfoort, NL) |
| 0,03 g | Mangan(II)acetat |
| 0,20 g | Parfüm |
| 95,16 g | Wasser |

### Beispiel 6

### Schaumfestiger mit starker Festigung

| | |
|---|---|
| 1,00 g | Trimethylolpropan-ethoxylat-20-triacrylat (Sartomer |
| | SR 415, Fa. Sartomer, Exton, PA) |
| 1,00 g | Bisphenol-A-ethoxylat-30-dimethacrylat (Sartomer SR 9036, |
| | Fa. Sartomer, Exton, PA) |
| 2,00 g | Vinylpyrrolidon/Methylaminoethylmethacrylat Copolymer |
| | (Copolymer 845, Fa. ISP, Wayne, NJ) |
| 0,45 g | Glyceryllaurat |
| 0,15 g | Parfüm |
| 0,16 g | Cetyltrimethylammoniumchlorid |
| 0,02 g | 2,2'-Azobis(2-amidino-propan)dihydrochlorid (V-50 der |
| | Fa. Wako, Osaka, JP) |
| 5,00 g | Propan/Butan (5,0 bar) |
| 14,95 g | Ethanol |
| 75,29 g | Wasser |

### Beispiel 7

### Schaumfestiger

| | |
|---|---|
| 2,00 g | Aromatisches Polyurethanacrylat (Laromer LR 8949, |
| | Fa. BASF, Ludwigshafen/Rh.) |
| 1,00 g | Aliphatisches Polyurethanacrylat (Laromer LR 8983, |
| | Fa. BASF, Ludwigshafen/Rh.) |
| 1,00 g | Vinylpyrrolidon/Methylaminoethylmethacrylat Copolymer |
| | (Copolymer 958, Fa. ISP, Wayne, NJ) |
| 0,20 g | 1,2-Propylenglykol |
| 0,17 g | Parfüm |
| 0,10 g | Cetyltrimethylammoniumchlorid |
| 6,00 g | Propan/Butan (5,0 bar) |
| 0,02 g | 2,2'-Azobis(N,N' dimethylenisobutyramidin) (VA-061 der |
| | Fa. Wako, Osaka, JP) |
| 18,66 g | Ethanol |
| 70,87 g | Wasser |

### Beispiel 8

### Schaumfestiger

| | |
|---|---|
| 2,00 g | Poly(ethylenglykol)-bis-(epoxypropylether) (Fa. Aldrich, |
| | Deisenhofen) |
| 2,00 g | Chitosan |
| 2,00 g | Polyvinylpyrrolidon (Luviskol K 30, Fa. BASF, Ludwigs- |
| | hafen/Rh.) |
| 0,20 g | 1,2-Propylenglykol |
| 0,17 g | Parfüm |
| 0,10 g | Cetyltrimethylammoniumchlorid |
| 6,00 g | Propan/Butan (5,0 bar) |
| 0,03 g | tert-Butylperoxybenzoat (in Form der 50%igen Lösung in |
| | Isododecan, Trigonox F-C50, Fa. Akzo Nobel Chemicals, |
| | Amersfoort, NL) |
| 69,04 g | Ethanol |
| 19,49 g | Wasser |

### Beispiel 9

### Pflegender Schaumfestiger

| | |
|---|---|
| 1,12 g | Polyurethanacrylatdispersion nach DE 19816527 A1, |
| | Formulierung I.1 |
| 1,12 g | Polyurethanacrylatdispersion nach DE 19816527 A1, |
| | Formulierung II.1 |
| 3,40 g | Vinylcaprolactam/Vinylpyrrolidon/Dimethylaminoethyl- |
| | methacrylat Terpolymer (Copolymer VC 713, Fa. ISP, |
| | Wayne, NJ) |
| 0,60 g | Ameisensäure |
| 0,60 g | Hydriertes Rizinusöl, ethoxyliert mit 40 Mol Ethylenoxid |
| 0,22 g | Decylpolyglucosid |
| 0,09 g | Cetyltrimethylammoniumchlorid |
| 0,20 g | Parfüm |
| 6,00 g | Propan/Butan (5,0 bar) |
| 87,77 g | Wasser |

### Beispiel 10

### Styling-Haarspray

| | |
|---|---|
| 1,14 g | Aminmodifiziertes Polyurethanacrylat (Laromer 8869, |
| | Fa. BASF, Ludwigshafen/Rh.) |
| 1,00 g | Oligoetheracrylat (Laromer 8967, Fa. BASF, Ludwigs- |
| | hafen/Rh.) |
| 1,00 g | Polyesteracrylat (PE 55 F, Fa. BASF, Ludwigshafen/Rh.) |
| 1,50 g | Octylacrylamid/Butylaminoethylmethacrylat/Methacrylat |
| | Copolymer (Amphomer 28-4910, Fa. National Starch, |
| | Bridgewater, NJ) |
| 0,15 g | Parfüm |
| 0,04 g | 2,5-Bis (tert-butylperoxy) -2,5-dimethyl-3-hexin (Trigonox |
| | 145-E85, Fa. Akzo Nobel Chemicals, Amersfoort, NL) |
| 0,02 g | Dimethyl-2,2'-azobis(isobutyrat) (V-601 der Fa. Wako, |
| | Osaka, JP) |
| 10,67 g | Butan (1,5 bar) |
| 33,33 g | Propan/Butan |
| 51,21 g | Ethanol |

### Beispiel 11

### Pumpspray

| | |
|---|---|
| 2,79 g | Polyesteracrylat (PE 55 W, Fa. BASF, Ludwigshafen/Rh.) |
| 0,30 g | Parfüm |
| 0,10 g | Dimethylsiloxan/Ethylenglykol Copolymer (Belsil DMC 6032, |
| | Fa. Wacker, Burghausen) |
| 0,02 g | tert-Butylhydroperoxid (in Form der 70% wäßrigen Lösung, |
| | Trigonox A-W70, Fa. Akzo Nobel Chemicals, Amersfoort, NL) |
| 11,53 g | Wasser |
| 85,28 g | Ethanol |

### Beispiel 12

### 80 % VOC-Haarspray

| | |
|---|---|
| 2,45 g | Aromatisches Polyurethanacrylat (Laromer LR 8983, |
| | Fa. BASF, Ludwigshafen/Rh.) |
| 4,00 g | Vinylacetat/Crotonsäure/Vinylpropionat Copolymer |
| | (Luviset CAP, Fa. BASF, Ludwigshafen/Rh.) |
| 0,20 g | Cyclopenta(dimethylsiloxan) |
| 0,15 g | Parfüm |
| 0,10 g | Bis(3,5,5-trimethylhexanoyl)peroxid (in Form der 50 % |
| | wäßrigen emulsion, Trigonox 36 W, Fa. Akzo Nobel |
| | Chemicals, Amersfoort, NL) |
| 13,20 g | Wasser |
| 40,00 g | Ethanol |
| 40,00 g | Dimethylether |

### Beispiel 13

### Pumpspray

| | |
|---|---|
| 2,79 g | Polyesteracrylat (PE 55 W, Fa. BASF, Ludwigshafen/Rh.) |
| 0,30 g | Parfüm |
| 0,20 g | Dimethylsiloxan/Ethylenglykol Copolymer (Belsil DMC 6032, |
| | Fa. Wacker, Burghausen) |
| 0,02 g | NIR-Initiator (Nr. **1**) |
| 11,53 g | Wasser |
| 85,28 g | Ethanol |

### Beispiel 14

### Pumpspray

| | |
|---|---|
| 0,50 g | Alkydharz (Alkydal F 300, Fa. Bayer, Leverkusen) |
| 1,00 g | Caprolactonacrylat (Sartomer SR 495, Fa. Sartomer, Exton, |
| | PA) |
| 0,80 g | Polyethylenglykoldiallylether (Fa. BASF, Ludwigs- |
| | hafen/Rh.) |
| 1,20 g | Dipentaerythritpentaacrylat (Sartomer SR 399 Fa. |
| | Sartomer, Exton, PA) |
| 0,30 g | Parfüm |
| 0,10 g | Dimethylsiloxan/Ethylenglykol Copolymer (Belsil DMC 6032, |
| | Fa. Wacker, Burghausen) |
| 0,1 g | tert-Butylhydroperoxid (in Form der 70% wäßrigen Lösung, |
| | Trigonox A-W70, Fa. Akzo Nobel Chemicals, Amersfoort, NL) |
| 0,05 g | Mangan(II)acetat |
| 11,53 g | Wasser |
| 85,28 g | Ethanol |

### Beispiel 15

### Festigendes Haarstylinggel

| | |
|---|---|
| 1,53 g | anorganisch-organisches Hybridprepolysiloxan aus |
| | Mercaptopropyltriethoxysilan und Salzsaure (nach |
| | DE 19822722, System 9) |
| 2,50 g | Polyvinylpyrrolidon |
| 2,10 g | Hydroxypropyl-Guar |
| 0,80 g | Hydriertes Rizinusöl, oxethyhert mit 45 Mol Ethylenoxid |
| 0,45 g | Natriumbenzoat |
| 0,30 g | Hydroxyethylcellulose |
| 0,20 g | Parfüm |
| 0,09 g | Natriumformiat |
| 0,05 g | Mica/Titanoxid/Zinnoxid-Pulver (Soloron® Silver Sparkle |
| | der Firma Merck, Deutschland) |
| 91,98 g | Wasser |

### Beispiele 16

### Haar-Cocktails

| | | |
|---|---|---|
| Phase A | 3,00 Gew.-% | Luvigel EM™ (BASF) |
| | 2,00 Gew.-% | Belsil DM 1000™ (Wacker) |
| | 3,00 Gew.-% | Belsil CM 1000™ (Wacker) |
| | 2,00 Gew.-% | Belsil PDM 200™ (Wacker) |
| | 2,00 Gew.-% | Belsil ADM 6057 E™ (Wacker) |
| | 0,50 Gew.-% | Belsil DMC 6031™ (Wacker) |
| | 1,00 Gew.-% | Macadamianußöl (Bsp. Huile de Macadamio von Wacker) |
| | 0,50 Gew.-% | Vitamin-E-Acetats™ (BASF) |
| | 1,00 Gew.-% | Cremophor RH 40™ (BASF) |
| | 0,40 Gew.-% | Parfümöl |
| | | |
| Phase B | 4,00 Gew.-% | Polymer gemäß Beispiel 14 |
| | 0,46 Gew.-% | 2-Amino-2-methylpropanol |
| | 0,10 Gew.-% | Euxyl K 100™ (Schulke & Mayr) |

ad 100,00 Wasser entmineralisiert

### Beispiel 17

### Hair Repair

| | |
|---|---|
| 6,00 % | Luviflex Silk™ (BASF) |
| 0,69 % | 2-Amino-2-methylpropanol |
| 0,20 % | Hydrolized Wheat Protein (Bsp. Cropesol W™ von Croda, Inc.) |
| 0,50 % | D-Panthenol USP™ (BASF) |
| 5,00 % | 1,2-Propylen GlycolUSP™ (BASF) |
| 10,00 % | Ethanol. |
| 77,61 % | Wasser entmineralisiert |

### Beispiel 18

### Hautcreme

Gemäß folgender Rezeptur wurde zunächst eine erfindungsgemäße Wasser/Öl-Cremeemulsion (Hautcreme A) hergestellt:

| | Zusatz | Gew.-% |
|---|---|---|
| Cremophor A 6 | Ceteareth-6 und Stearylalkohol | 2,0 |
| Chremophor A 25 | Ceteareth-25 | 2,0 |
| Lanette O | Cetearylalkohol | 2,0 |
| Imwitor 960 K | Glycerylstearat SE | 3,0 |
| Paraffinöl | | 5,0 |
| Jojobaöl | | 4,0 |
| Luvitol EHO | Cetearyloctanoat | 3,0 |
| ABIL 350 | Dimethicone | 1,0 |
| Amerchol L 101 | Mineralöl und Lanolinalkohol | 3,0 |
| Veegum Ultra | Magnesiumaluminiumsilikat | 0,5 |
| 1,2-Propylenglykol | Propylenglykol | 5,0 |
| Abiol | Imidazolindinyl-Harnstoff | 0,3 |
| Phenoxyethanol | | 0,5 |
| D-Panthenol USP | | 1,0 |
| Polymer (Luviskol VA 63, BASF)) | | 0,5 |
| Wasser | | ad 100 |

### Beispiel 19

### Duschgel

Gemäß folgender Rezeptur wurde zunächst eine erfindungsgemäße Duschgel-Formulierung (Duschgel A) hergestellt:

| | Zusatz | Gew.-% |
|---|---|---|
| Texapon NSO | Natriumlaurethsulfat | 40,0 |
| Tego Betain L7 | Cocamidopropylbetain | 5,0 |
| Plantacare 2000 | Decylglucosid | 5,0 |
| Parfüm | | 0,2 |
| Polymer gemäß Beispiel 15 | | 0,2 |
| Euxyl K 100 | Benzylalkohol, Methylchlorisothiazolinon, Methylisothiazolinon | 0,1 |
| D-Panthenol USP | | 0,5 |
| Citronensäure (pH 6-7) | | q.s. |
| NaCl | | 2,0 |
| Wasser | | ad 100 |

### Beispiel 20

### Feuchthalteformulierung

### Formulierung A

| | Zusatz | | Gew.-% |
|---|---|---|---|
| a) | Cremophor A6 | Ceteareth-6 und Stearylalkohol | 2,0 |
| | Cremophor A25 | Ceteareth-25 | 2,0 |
| | Paraffinöl (dickflüssig) | | 10,0 |
| | Lannette O | Cetearylalkohol | 2,0 |
| | Stearinsäure | | 3,0 |
| | Nip-Nip | Methylparaben/Propylparaben 70:30 | 0,5 |
| | Abiol | Imidazoldinyl-Harnstoff | 0,5 |
| b) | Polymer (Hypermer B 246, ICI)) | | 3,0 |
| | Wasser | | ad 100 |

Beide Phasen wurden auf 80°C erhitzt, Phase a) wurde in b) eingerührt, homogenisiert und kaltgerührt und anschließend mit 10%iger wässriger NaOH-Lösung auf pH 6 eingestellt.

### Beispiel 21

### O/W Creme zur Hautfeuchthaltung

| Zusatz | Gew.-% |
|---|---|
| Glycerinmonostearat | 2,0 |
| Cetylalkohol | 3,0 |
| Paraffinöl, subliquidum | 15,0 |
| Vaseline | 3,0 |
| Caprylcaprinsäuretriglycerid | 4,0 |
| Octyldodecanol | 2,0 |
| Hydriertes Kokosfett | 2,0 |
| Cetylphosphat | 0,4 |
| Polymer (Beispiel 18) | 3,0 |
| Glycerin | 3,0 |
| Natriumhydroxid | q.s. |
| Parfümöl | q.s. |
| Konservierungsmittel | q.s. |
| Wasser | ad 100 |

### Beispiel 22

### O/W-Lotion

| Zusatz | Gew.-% |
|---|---|
| Stearinsäure | 1,5 |
| Sorbitanmonostearat | 1,0 |
| Sorbitanmonooleat | 1,0 |
| Paraffinöl, subliquidum | 7,0 |
| Cetylalkohol | 1,0 |
| Polydimethylsiloxan | 1,5 |
| Glycerin | 3,0 |
| Polymer (Beispiel 20) | 0,5 |
| Parfümöl | q.s. |
| Konservierungsmittel | q.s. |
| Wasser | ad 100 |

### Beispiel 23

### W/O-Creme

| Zusatz | Gew.-% |
|---|---|
| PEG-7-hydriertes Ricinusöl | 4,0 |
| Wollwachsalkohol | 1,5 |
| Bienenwachs | 3,0 |
| Triglycerid, flüssig | 5,0 |
| Vaseline | 9,0 |
| Ozokerite | 4,0 |
| Paraffinöl, subliquidum | 4,0 |
| Glycerin | 2,0 |
| Polymer (Beispiel 20) | 2,0 |
| Magnesiumsulfat^{*}7H₂O | 0,7 |
| Parfümöl | q.s. |
| Konservierungsmittel | q.s. |
| Wasser | ad 100 |

### Beispiel 24

### Hydrogel zur Hautpflege

| Zusatz | Gew.-% |
|---|---|
| Polymer (Herstellungsbeispiel 19) | 3,0 |
| Sorbit | 2,0 |
| Glycerin | 3,0 |
| Polyethylenglycol 400 | 5,0 |
| Ethanol | 1,0 |
| Parfümöl | q.s. |
| Konservierungsmittel | q.s. |
| Wasser | ad 100 |

### Beispiel 25

### Hydrodispersionsgel

| Zusatz | Gew.-% |
|---|---|
| Polymer (Herstellungsbeispiel 19) | 3,0 |
| Sorbit | 2,0 |
| Glycerin | 3,0 |
| Polyethylenglycol 400 | 5,0 |
| Triglycerid, flüssig | 2,0 |
| Ethanol | 1,0 |
| Parfümöl | q.s. |
| Konservierungsmittel | q.s. |
| Wasser | ad 100 |

### Beispiel 26

### Flüssige Seife

| Zusatz | Gew.-% |
|---|---|
| Kokosfettsäure, Kaliumsalz | 15 |
| Kaliumoleat | 3 |
| Glycerin | 5 |
| Polymer (Herstellungsbeispiel 14) | 2 |
| Glycerinstearat | 1 |
| Ethylenglycoldistearat | 2 |
| Spezifische Zusätze, Komplexierungsmittel, Duftstoffe | q.s. |
| Wasser | ad 100 |

### Beispiel 27

### Body Care Cream

| | Zusatz | Gew.-% |
|---|---|---|
| Cremophor A6 | Ceteareth-6 und Stearylalkohol | 2,0% |
| Cremophor A 25 | Ceteareth-25 | 2,0% |
| Grape (Vitis Vinifera) | Seed oil | 6,0% |
| Glycerylstearat SE | | 3,0% |
| Cetearylalkohol | | 2,0% |
| Dimethicon | | 0,5% |
| Luvitol EHO | Cetearyloctanoat | 8,0% |
| Oxynex 2004 | Propylenglycol, BHT, Ascorbylpalmitat, Glycerylstearat, Citronensäure | 0,1% |
| Konservierungsmittel | | q.s. |
| 1,2-Propylenglykol USP | | 3,0% |
| Glycerin | | 2,0% |
| EDTA BD | | 0,1% |
| D-Panthenol USP | | 1,0% |
| Wasser | | ad 100 |
| Polymer (Beispiel 20) | | 1,5% |
| Tocopherylacetat | | 0,5% |

Die Formulierung wies einen pH-Wert von 6,8 auf. Die Viskosität (Brookfield RVT, 23°C) betrug 32000 mPas.

### Beispiel 28

### Maskara (Wimperntusche)

| | | |
|---|---|---|
| Phase A | 1,50 % | Cremophor A6™ (BASF) |
| | 1,50 % | Cremophor A25™ (BASF) |
| | 2,00 % | Stearinsäure (Bsp. Emersol 120™ von Kenkel) |
| | 3,00 % | Imwitor 960 K™ (Hüls AG) |
| | 3,00 % | Softisan 100™ (Hüls AG) |
| | 1,50 % | Luvigel EM™ (BASF) |
| | 10,00 % | Dow Corning 345™ (Dow Corning) |
| Phase B | 4,00 % | Luviflex Silk™ (BASF) |
| | 0,46 % | 2-Amino-2-methylpropanol |
| | 0,30 % | Germal 115™ (Sutton) |
| | 72,24 % | Wasser entmineralisiert |
| Phase C | 0,50 % | Phenoxyethanol (Bsp. Phenoxetol™ von Nipa-Hardwicke) |

### Beispiel 29

### Make-up

| | | | | |
|---|---|---|---|---|
| A | 2.0 | Cremophor A 6 | (1) | Ceteareth-6, Stearyl Alcohol |
| | 2.0 | Cremophor A 25 | (1) | Ceteareth-25 |
| | 4.0 | Cetylstearylalkohol | | Cetearyl Alcohol |
| | 6.0 | Luvitol EHO | (1) | Cetearyl Octanoate |
| | | | | |
| B | 4.0 | 1,2-Propylenglykol USP | (1) | Propylene Glycol |
| | 2.0 | D-Panthenol 50 P | (1) | Panthenol, Propylene Glycol |
| | 0.5 | Wirkstoffkonzentration Polymer | | |
| | q.s. | Neutralisationsmittel (falls erforderlich) | | |
| | q.s. | Konservierungsmittel | | |
| | ad 100 | Wasser dem. | | |
| | | | | |
| C | q.s. | Vitamine | | |
| | q.s. | Parfümöl | | |

### Herstellung

Die Phasen A und B getrennt auf ca. 80°C erwärmen, Phase A in Phase B einrühren und homogenisieren. Unter Rühren auf ca. 40°C abkühlen, Phase C einarbeiten und nochmals homogenisieren.

### Beispiel 30

### Sonnenschutzemulsion

| | | | | |
|---|---|---|---|---|
| | 1.00 | Cremophor A 6 | (1) | Ceteareth-6, Stearyl Alcohol |
| | 2.00 | Cremophor A 25 | (1) | Ceteareth-25 |
| | 3.00 | Glycerinmonostearat | (44) | Glyceryl Stearate |
| | 2.00 | Cetylstearylalkohol | (27) | Cetearyl Alcohol |
| | 2.00 | Luvitol EHO | (1) | Cetearyl Octanoate |
| | 1.00 | Uvinul T 150 | (1) | Octyl Triazone |
| | 5.00 | Uvinul MC 80 | (1) | Octyl Methoxycinnamate |
| | 3.00 | Uvinul MBC 95 | (1) | 4-Methylbenzylidene Camphor |
| | 5.00 | Z-Cote | (1) | Zinc Oxide |
| | 7.00 | Isopropylmyristat | (27) | Isopropyl Myristate |
| | | | | |
| B | 0.20 | Edeta BD | (1) | EDTA |
| | 0.50 | D-Panthenol USP | (1) | Panthenol |
| | 5.00 | 1,2-Propylenglykol USP | (1) | Propylene Glycol |
| | 7.50 | Luviquat Care | (1) | Polyquaternium-44 |
| | q.s. | Konservierungsmittel | | |
| | 54.50 | Wasser dem. | | Aqua dem. |
| | | | | |
| C | 0.30 | Keltrol T | (66) | Xanthan Gum |
| | | | | |
| D | 1.00 | Vitamin E-Acetat | (1) | Tocopheryl Acetate |
| | q.s. | Parfümöl | | |

| Lieferanten | |
|---|---|
| (1) | BASF Aktiengesellschaft |
| (27) | Cognis Deutschland GmbH |
| (44) | Th. Goldschmidt AG |
| (66) | Kelco International GmbH |

## Patentansprüche

1. Verfahren zur Behandlung eines kosmetischen Mittels, dadurch gekennzeichnet, daß das kosmetische Mittel vor, während oder nach der Applikation mit NIR-Strahlung behandelt wird.

2. Verfahren gemäß Anspruch 1, dadurch gekennzeichnet, daß das Emissionsmaximum der NIR-Strahlung im Wellenlängenbereich von 600 bis 1500 nm liegt.

3. Verfahren gemäß einem der Ansprüche 1 oder 2, dadurch gekennzeichnet, daß das kosmetische Mittel zur Behandlung der Haut, der Haare und/oder der Nägel eingesetzt wird.

4. Verfahren gemäß einem der Ansprüche 1 bis 3, dadurch gekennzeichnet, daß das kosmetische Mittel filmbildende oder permeationshemmende Eigenschaften aufweist.

5. Verfahren gemäß einem der Ansprüche 1 bis 4, dadurch gekennzeichnet, daß das kosmetische Mittel wenigstens ein Polymer enthält.

6. Verfahren gemäß einem der Ansprüche 1 bis 5, dadurch gekennzeichnet, daß durch Wirkung der NIR-Strahlung wenigstens ein Polymer gebildet wird und/oder das Molekulargewicht wenigstens eines Polymers erhöht wird und/oder die Eigenschaften des Polymers verändert werden.

7. Verfahren gemäß einem der Ansprüche 1 bis 6, dadurch gekennzeichnet, daß das Polymer ein Poly(meth)acrylat, Polyamid, Polyester, Polyether, Polyurethan, Poly-N-vinyllactam, Polyolefin, Polyvinylester, Polysiloxan oder ein Copolymer enthaltend Wiederholungseinheiten der vorgenannten Polymere ist.

8. Verfahren nach einem der Ansprüche 1 bis 7, dadurch gekennzeichnet, daß das Polymer wenigstens eine Wiederholungseinheit aufweist, die aus Verbindungen der Gruppe Methylmethacrylat, tert.-Butylacrylat, 2-Butylmethacrylat, Styrol, N-tert.-Butylacrylamid, Vinylacetat, Vinylpropionat, Vinylcaprolactam, Vinylpyrrolidon, Vinylimidazol und N-Methylvinylimidazoliniumsalz, Ethylacrylat, Methylacrylat, Hydroxyethylacrylat, n-Butylacrylat, Lauryl (meth) acrylat, Stearyl(meth)acrylat, Vinylneodecanoat, Acrylsäure, Methacrylsäure, Crotonsäure hervorgeht.

9. Verfahren nach einem der Ansprüche 1 bis 8, dadurch gekennzeichnet, daß das Polymer ein Polyelektrolyt ist.

10. Verfahren nach einem der Ansprüche 1 bis 9, dadurch gekennzeichnet, daß das Polymer durch Polymerisation, Polyaddition oder Polykondensation oder eine Kombination dieser Verfahren gebildet wird.

11. Verfahren nach einem der Ansprüche 1 bis 10, dadurch gekennzeichnet, daß die Intensität der Strahlung auf den Zweck der Behandlung abgestimmt werden kann.

12. Verfahren nach einem der Ansprüche 1 bis 11, dadurch gekennzeichnet, daß die Strahlung von mehreren Strahlungsquellen erzeugt wird, wobei die Art und Intensität der Strahlungsquellen gleich oder unterschiedlich sein kann.

13. Verfahren nach einem der Ansprüche 1 bis 12, dadurch gekennzeichnet, daß die Strahlungsquellen äquidistant zum Kopf positioniert sind.

14. Verfahren nach einem der Ansprüche 1 bis 13, dadurch gekennzeichnet, daß die Strahlungsquellen äquidistant zueinander positioniert sind.

15. Kosmetisches Mittel, erhältlich durch Bestrahlung mit NIR-Strahlung.

16. Kosmetisches Mittel gemäß Anspruch 15, dadurch gekennzeichnet, daß das kosmetische Mittel wenigstens eine ethylenisch ungesättigte Verbindung, die sowohl niedermolekular wie auch hochmolekular sein kann, enthält.

17. Kosmetisches Mittel gemäß Anspruche 15 oder 16, dadurch gekennzeichnet, daß das Emissionsmaximum der NIR-Strahlung im Wellenlängenbereich von 600 bis 1500 nm liegt.

18. Kosmetisches Mittel, gemäß einem der Ansprüche 15 bis 17, dadurch gekennzeichnet, daß unter Einwirkung von NIR-Strahlung mindestens ein Polymer gebildet wird.

19. Kosmetisches Mittel gemäß einem der Ansprüche 15 bis 18, dadurch gekennzeichnet, daß das kosmetische Mittel wenigstens einen Initiator, Farbstoff oder Katalysator, der die Wirkung der Strahlung ermöglicht, beschleunigt oder verstärkt, enthält.

20. Kosmetisches Mittel gemäß einem der Ansprüche 15 bis 19, dadurch gekennzeichnet, daß das kosmetische Mittel wenigstens einen Initiator für eine radikalische Polymerisationen enthält.

21. Kosmetisches Mittel gemäß einem der Ansprüche 15 bis 20, dadurch gekennzeichnet, daß das kosmetische Mittel wenigstens eine anorganische Verbindung mit einer O-O-Bindung, und optional Reduktionsmittel enthält.

22. Kosmetisches Mittel gemäß einem der Ansprüche 15 bis 21, dadurch gekennzeichnet, daß das kosmetische Mittel wenigstens eine nieder- oder hochmolekulare Verbindung enthält, die wenigstens eine und bevorzugt zwei bis acht Funktionalitäten aus der Gruppe -OH, -NHR, -COOR, -C(H)=O, Epoxid, -NCO, aufweist, wobei R Wasserstoff oder ein beliebiger organischer Rest ist.

23. Kosmetisches Mittel gemäß einem der Ansprüche 15 bis 22, dadurch gekennzeichnet, daß das kosmetische Mittel Verbindungen ausgewählt aus der Gruppe (meth)acrylatfunktionelle (Meth)acrylcopolymere, Polyether(meth) acrylate, Polyester(meth)acrylate, ungesättigte Polyester, Epoxy(meth)acrylate, Urethan(meth)acrylate, Amino(meth)acrylate, Melamin(meth)acrylate, Silikon(meth)acrylate neben üblichen Zusätzen enthält.

24. Kosmetisches Mittel gemäß einem der Ansprüche 15 bis 23, enthaltend neben übliche Zusätzen ein weiteres anionisches, kationisches, neutrales oder betaines Polymer, ein Biopolymer oder ein Präpolymer.

25. Verwendung eines kosmetischen Mittels, gemäß einem der Ansprüche 15 bis 24 zur Behandlung der Haut, der Haare oder der Nägel.

26. Verwendung des kosmetischen Mittels gemäß Anspruch 25, zur Behandlung der Haare.

27. Verwendung des kosmetischen Mittels gemäß einem der Ansprüche 25 oder 26, dadurch gekennzeichnet, daß das kosmetische Mittel ausgewählt ist aus der Gruppe der Dauerwellpräparate, der Haarkuren, Haarlotionen, Haarspülungen, Haaremulsionen, Spitzenfluids, Egalisierungsmittel für Dauerwellen, Hot-Oil-Treatment-Präparate, Conditioner, Festigerlotionen, Shampoos, Haarfärbemittel, Haarsprays, Schaumfestiger, Haarmousse, Haargel, Mittel zur Behandlung von Schuppen und Haarausfall sowie Haarwuchsmittel.

28. Verwendung des kosmetischen Mittels gemäß Anspruch 25 zur Behandlung der Nägel.

29. Verwendung des kosmetischen Mittels gemäß Anspruch 28, dadurch gekennzeichnet, daß das Mittel zur Behandlung der Nägel ausgewählt ist aus der Gruppe der Nagelpflegemittel sowie der Nagellacke.

30. Verwendung des kosmetischen Mittels gemäß Anspruch 25, dadurch gekennzeichnet, daß das Mittel ausgewählt ist aus den kosmetischen Mitteln zur Behandlung der Haut und der Wundbehandlung.

31. Verwendung des kosmetischen Mittels gemäß Anspruch 30, dadurch gekennzeichnet, daß das Mittel zur Behandlung der Haut ausgewählt ist aus der Gruppe der W/O- oder O/W-Hautcremes, Tag- und Nachtcremes, Augencremes, Gesichtscremes, Antifaltencremes, Feuchthaltecremes, Bleichcremes, Vitamincremes, Hautreinigungsmitteln, Babypflegemitteln sowie der dekorativen Kosmetik.

32. Verwendung des kosmetischen Mittels gemäß einem der Ansprüche 25 bis 31, dadurch gekennzeichnet, daß nach Bestrahlung mit NIR-Strahlung eine Glastemperatur des Mittels größer 20°C resultiert.
